# EUROPEAN PATENT SPECIFICATION

(11) **EP 2 758 069 B1**
(45) Date of publication and mention of the grant of the patent: **17.05.2017**
(21) Application number: 12762585.3
(22) Date of filing: 21.09.2012
(51) Int. Cl.: A61K 38/19, A61K 48/00, C12Q 1/68, G01N 33/50, G01N 33/68

(54) **METHOD TO PREDICT THE PRESENCE OF INFLAMMATION OR ITACONIC ACID, IRG1 AND/OR PROTEIN IRG1 IN A SUBJECT AND PHARMACEUTICAL COMPOSITION FOR TREATING OR PREVENTING INFLAMMATION**
VERFAHREN ZUR VORHERSAGE DES VORHANDENSEINS VON ENTZÜNDUNGEN ODER ITACONSÄURE, IRG1 UND/ODER DES PROTEINS IRG1 IM KÖRPER EINES PATIENTEN UND PHARMAZEUTISCHE ZUSAMMENSETZUNG ZUR BEHANDLUNG ODER PRÄVENTION VON ENTZÜNDUNGEN
PROCÉDÉ DE PRÉVISION DE LA PRÉSENCE D'UNE INFLAMMATION OU D'ACIDE ITACONIQUE, D'IRG1 ET/OU D'UNE PROTÉINE IRG1 CHEZ UN SUJET ET COMPOSITION PHARMACEUTIQUE POUR LE TRAITEMENT OU LA PRÉVENTION DE L'INFLAMMATION

(30) Priority: 23.09.2011 LU 91877
(43) Date of publication of application: 30.07.2014
(73) Proprietor: Université du Luxembourg, 1511 Luxembourg (LU)
(72) Inventor: HILLER, Karsten, 66663 Besseringen (DE); MICHELUCCI, Alessandro, L-4362 Esch-sur-Alzette (LU); CORDES, Thekla, L-1630 Luxembourg (LU); WEGNER, Andre, 54292 Trier (DE); GHELFI, Jenny, L-4362 Esch-sur-Alzette (LU); BALLING, Rudi, L-1867 Howald (LU)
(74) Representative: Schiweck, Weinzierl & Koch Patentanwälte Partnerschaft mbB
(86) International application number: PCT/EP2012/068682
(87) International publication number: WO 2013/041692

(56) References cited:
- BASLER TINA ET AL: "Mycobacterium paratuberculosis, Mycobacterium smegmatis, and lipopolysaccharide induce different transcriptional and post-transcriptional regulation of the IRG1 gene in murine macrophages", JOURNAL OF LEUKOCYTE BIOLOGY, FEDERATION OF AMERICAN SOCIETIES FOR EXPERIMENTAL BIOLOGY, US, vol. 79, no. 3, 1 March 2006 (2006-03-01), pages 628-638, XP009157223, ISSN: 0741-5400, DOI: 10.1189/JLB.0905520
- DEGRANDI DANIEL ET AL: "The Proinflammatory Cytokine-Induced IRG1 Protein Associates with Mitochondria", JOURNAL OF INTERFERON AND CYTOKINE RESEARCH, MARY ANN LIEBERT, NEW YORK, NY, US, vol. 29, no. 1, 1 January 2009 (2009-01-01), pages 55-67, XP009157217, ISSN: 1079-9907, DOI: 10.1089/JIR.2008.0013 [retrieved on 2008-11-17]
- WEI XIAO ET AL: "Expression profile of human immune-responsive gene 1 and generation and characterization of polyclonal antiserum", MOLECULAR AND CELLULAR BIOCHEMISTRY, KLUWER ACADEMIC PUBLISHERS, BO, vol. 353, no. 1 - 2, 20 March 2011 (2011-03-20), pages 177-187, XP019911173, ISSN: 1573-4919, DOI: 10.1007/S11010-011-0784-7
- STRELKO CHERYL L ET AL: "Itaconic acid is a mammalian metabolite induced during macrophage activation", JOURNAL OF THE AMERICAN CHEMICAL SOCIETY, AMERICAN CHEMICAL SOCIETY, UNITED STATES, vol. 133, no. 41, 15 September 2011 (2011-09-15), pages 16386-16389, XP009157219, ISSN: 1520-5126

## Description

The invention is mainly based on the use of itaconic acid, IRG1 and protein IRG1 level in mammalian cells as biomarker for diagnosing inflammation. The invention is directed the ability of mammalian cells to produce itaconic acid in therapy for preventing and/or treating inflammation.

Inflammation is part of the biological response of tissues to protect the organism and to remove injurious stimuli such as pathogens, damaged cells or irritants, and to initiate the healing process. Inflammation can be caused by infection by a microorganism. The identification of biomarkers and/or products able to diagnose, prevent, or treat inflammation is of interest within the health field.

Itaconic acid, also known as methylene succinic acid (C₅H₆O₄), is a soluble unsaturated dicarboxylic acid mainly produced from sugars by several fungi. It is used worldwide in industry as monomer or co-monomer in the manufacture of plastics, resins, synthetic fibers, paints, etc. It is also used as an acidulant and for the pH adjustment of food. Itaconic acid is naturally produced in *Aspergillus* and especially in *Aspergillus terreus* which shows high production rate. Biotechnical production of itaconic acid through fungal fermentation is well documented and is described for example in "Biotechnological production of itaconic acid and its biosynthesis in Aspergillus terreus" - Okabe et al. Appl Microbiol Biotechnol (2009) 84: 597-606.

The itaconic acid biosynthesis route in *A. terreus* has not yet been fully established. One of the problems encountered is that the pathway towards itaconic acid occurs in two compartments, in the cytosol and in the mitochondria. The pathway was studied and described in:
- "A clone-based transcriptomics approach for the identification of genes relevant for itaconic acid in Aspergillus" - Li et al. Fungal Genetics and biology 48 (2011) 206-611;
- "The subcellular organization of itaconate biosynthesis in Aspergillus terreus" - Jaklitsch et al. Journal of General Microbiology (1991), 137, 533-539.
- "Itaconate Biosynthesis in Aspergillus terreus" - Bonnarme et al. Journal of bacteriology, June 1995, p.3575 - 3578.

Briefly, in the mitochondria, citrate is converted into *cis*-aconitate in the tricarboxylic acid cycle (TCA). *Cis*-aconitate is being transported from mitochondria into cytosol and then decarboxylated to itaconate by the *cis*-aconitic acid decarboxylase (CAD).

The present invention shows itaconic acid production in mammalian subjects such as mouse and, in particular, in mouse macrophage and microglia cells. The present invention also shows itaconic acid production in human cells and in particular in human immune cells such as human macrophages. From prior art, itaconic acid was not known to play a role in mammalian metabolism. The invention shows that the intracellular itaconic acid level in mammalian cells is increased in response to inflammation. Therefore, a first aspect of the invention is related to the use of itaconic acid as a biomarker of inflammation in mammalian cells, and in particular in macrophage and microglia cells. A second aspect of the invention is related to a method for identifying a compound candidate for pharmacological agent useful in the treatment of inflammation using determination of the itaconic acid levels in cells.

Under normal physiological conditions, macrophages and microglia cells, respectively in tissues and in the central nervous system (CNS), maintain homeostasis and respond rapidly to perturbations in the local environment. The classic activation, which can be induced by in vitro culture of macrophages with lipopolysaccharide (LPS), is associated with high microbicidal activity and cytokines production. LPS is known to be a major component of the outer membrane of gram-negative bacteria and a potent and pleiotropic stimulus that dramatically enhances the inflammatory potential and performance of macrophages.

Among others, the murine pro-inflammatory cytokine-induced gene 1 (Immune-responsive gene 1 or IRG1) protein has been demonstrated to be highly up-regulated in LPS-stimulated macrophage. The IRG1 message following LPS exposure is disclosed in "Cloning and analysis of gene regulation of a novel LPS-inducible cDNA" - Lee et al. Immunogenetics (1995) 41: 263-270. This document also discloses that IRG1 is mapped to mouse chromosome 14. In "different Neurotropic Pathogens Elicit Neurotoxic CCR9- or Neurosupportive CXCR3-Expressing Microglia" - Li et al. Journal of immunology 2006; 177; 3644-3656; IRG1 is described to be a potential therapeutic target for gene therapy of some neurodegenerative and neuroinflammatory disease. However, its function is still unknown from prior art.

The present invention shows that the function of conversion of *cis*-aconitate to itaconic acid in mammalian cells can be assigned to IRG1. The present invention identifies the enzyme catalyzing the production of itaconic acid from cis-aconitate in mammalian cells, and demonstrates that the gene coding for this enzyme is known as immune response gene 1 (IRG1). Therefore, a third aspect of the invention is related to the use of itaconic acid as a biomarker of the presence of IRG1 and/or protein encoded by IRG1 in mammalian cells upon LPS exposure. A fourth aspect of the invention is to assess production of itaconic acid by determination of the presence, level and/or expression level of IRG1 and/or protein encoded by IRG1 in cells.

Moreover, it has been shown in the following documents that itaconic acid inhibited the isocitrate lysase, and in consequence the glyoxylate cycle:
- "Glyoxylate Bypass Enzymes in Yersinia Species and Multiple Forms of Isocitrate Lyase in Yersinia pestis" - Hillier and Charnetzky. Journal of bacteriology, Jan. 1981, p. 452-458.
- "Mycobacterium tuberculosis isocitrate lyases 1 and 2 are joinly required for in vivo growth and virulence"- Munoz-Elias and McKinney. Nature Medicine, vol. 11, number 6, June 2005, pages 638-644)

Isocitrate Lyase is the key enzyme of the glyoxylate shunt that is mobilized when bacteria are grown on fatty acids as the limiting carbon source. The glyoxylate cycle, a variation of the TCA cycle, is an anabolic metabolic pathway occurring in plants, bacteria, protists, fungi and several microorganisms such as E. coli. As the glyoxylate cycle is unique to prokaryotes, lower eukaryotes and plants, its inhibition affects the growth of these organisms.

The invention shows that cells produce itaconic acid in response to infection as natural antibiotic. Therefore, a further aspect of the invention is related to the use of IRG1 in gene therapy to induce the production of itaconic acid in cells. Another aspect of the invention is related to the use of a vector containing IRG1 as a pharmaceutical agent for treatment of inflammation.

### Summary of the invention

The invention is based, at least in part, on the following discoveries by the inventors:
- Mouse macrophages and microglia cells are able to produce itaconic acid under LPS inflammation.
- Human macrophages are able to produce itaconic acid under LPS inflammation.
- Macrophage and microglia cells show pro-inflammatory condition when producing itaconic acid.
- IRG1 up regulation is associated with the production of the itaconic acid.
- The protein encoded by IRG1 is the enzyme catalyzing the production of itaconic acid from *cis*-aconitate in mammalian cells.
- Macrophage and microglia cells produce itaconic acid as an immune response to inflammation as itaconic acid shows antimicrobial activity.

### Detailed description of the invention

Before further description of the invention, certain terms employed in the specification, example and appended claims are, for convenience, collected there.

### Definitions

As used herein "microglia cells" refer to resident antigen-presenting cells within the central nervous system (CNS) and they serve immune-like functions in protecting brain against injury and invading pathogens.

As used herein "macrophages" refer to cells produced by differentiation of monocytes in tissues. Macrophages function in both non-specific defense as well as help initiate specific defense mechanisms. Their role is to phagocytose cellular debris and pathogens, either as stationary or as mobile cells. They also stimulate lymphocytes and other immune cells to respond to pathogens. They are specialized *phagocytic* cells that attack foreign substances, infectious microorganism and cancer cells through destruction and ingestion.

As used herein "inflammation" refers the biological response of tissues to harmful stimuli such as pathogens, damaged cells or irritant.

As used herein "pathogens" are microorganisms such as virus, bacteria, prion or fungus.

As used herein "pro-inflammatory condition" refers to the activated state of macrophages or microglia cells.

As used herein, the term "antibody" encompasses not only intact polyclonal or monoclonal antibodies, but also fragments thereof, fusion protein comprising an antibody portion, single chain antibodies, multispecific antibodies (e.g., bispecific antibodies) and any other modified configuration of the immunoglobin molecule that comprise an antigen recognition site of the required specificity. An antibody includes an antibody of any class such as IgG, IgA or IgM (or subclass thereof), and the antibody needs not to be of any particular class.

As used herein, the term "labeled", with regard to the probe or antibody is intended to encompass direct labeling of the probe, primer or antibody by coupling (i.e. physically linking) a detectable substance to the probe or antibody, as well as indirect labeling of the probe or antibody by reactivity with another reagent that is directly labeled. Example of indirect labeling include detection of a primary antibody using a fluorescently labeled secondary antibody and end-labeling of a DNA probe with biotin such that it can be detected with fluorescently labeled streptavidin.

As used herein, the term "specific fragments" of a protein or a polypeptide refers to a particular fragment of an amino acid sequence having at least one of the functional characteristic or properties of the complete protein or polypeptide, notably in that it is capable of being recognized by a specific antibody and/or that the expression level of such protein or polypeptide is correlated to the expression level of the complete or partial IRG1 expressed.

As used herein, the term "specific fragments" of mRNA or cDNA transcribed from IRG1 refers to a particular fragment of an oligonucleotide sequence capable of hybridizing with at least one set of nucleic probe specific to IRG1 or to be amplified with at least one set of primers specific to IRG1.

### Continued description of the invention

In one aspect, the invention features the use of the absence, presence and/or level of itaconic acid in mammalian cells as biomarker for inflammation. If the mammalian cells are selected from the group comprising macrophages and microglia cells, the absence, presence and/or level of itaconic acid can be used as a biomarker of the pro-inflammatory condition of these cells.

Therefore, the invention is related to a method for detecting inflammation in a subject, comprising the step of determining the presence and/or the level of itaconic acid in a biological sample isolated from said subject wherein the presence and/or level of itaconic acid is indicative of the presence of inflammation.

In a preferred embodiment of the invention, inflammation is the biological response to pathogens or infectious agent, preferably to bacteria.

In a preferred embodiment, the presence of inflammation is indicative of the subject being infected by pathogens or infectious agent.

The invention is also related to a method for detecting pro-inflammatory condition in macrophages and/or microglia cells of a subject comprising the step of determining the presence and/or the level of itaconic acid in a biological sample isolated from said subject wherein the presence and/or the level of itaconic acid is indicative of a pro-inflammatory condition.

The invention is further related to a method to determine the ability of a subject, with preference a human subject, to react to inflammation, comprising the step of:
a. providing a biological sample isolated from said subject
b. contacting said biological sample with lipopolysaccharide
c. determining the presence and/or the level of itaconic acid in a biological sample,
wherein the presence and/or the level of itaconic acid is indicative of the ability of the subject to react to inflammation.

The above methods are preferably conducted *in vitro.* The methods of the invention involve lysis of the cells in the biological sample as itaconic acid is an intracellular metabolite. With preference the subject is mammal. The mammal subject can be non-human mammal subject such as mouse. The mammal subject can be human.

In another aspect the invention features the use of the absence, presence and/or level of itaconic acid in mammal cells under inflammation as biomarker of the presence of IRG1 and/or the protein encoded by IRG1.

The invention relates to a method for predicting the presence of IRG1 and/or protein encoded by IRG1 in a subject, comprising the steps of:
a- providing a biological sample isolated from said subject
b- determining the presence and/or the level of itaconic acid in a biological sample,
wherein the presence and/or the level of itaconic acid is indicative of the presence of IRG1 and/or of the protein encoded by IRG1, in at least a part of the cells of said biological sample. With preference, the biological sample of step (a) is contacted with lipopolysaccharide in order to initiate production of itaconic acid before the step (b) of determining the presence and/or the level of itaconic acid in the biological sample.

The above method is preferably conducted *in vitro.* The method of the invention involves lysis of the cells after a predetermined time in the biological sample as itaconic acid is an intracellular metabolite. The predetermined time range to about 1 to 8 hours after LPS treatment, with preference the predetermined time range to about 3 to 6 hours, with preference the predetermined time is 6 hours. With preference the subject is mammal. The mammal subject can be non-human mammal subject such as mouse. The mammal subject can be human.
In another aspect, the invention features the use of the absence, presence and/or level of itaconic acid in mammal cells as biomarker for inflammation in a method for identifying a compound candidate for pharmacological agent useful in the treatment of inflammation. With preference mammal cells are macrophage and/or microglia cells. Here disclosed is a method for identifying a compound candidate for pharmacological agent useful in the treatment of inflammation comprising the steps of:
a- contacting a non-human mammal subject, with preference a mouse subject, presenting inflammation with a candidate pharmacological agent;
b- determining the level of itaconic acid in a biological sample isolated from said subject;
such that the decrease in the test amount of level of itaconic acid indicates that the candidate pharmacological agent is a potential compound for a pharmaceutical agent useful in the treatment of inflammation.
In a preferred embodiment, a preliminary step of determination of itaconic acid level in the biological sample isolated from the subject is conducted before the step of contacting the subject with a candidate pharmacological agent. In the above method the steps of determining the level of itaconic acid is preferably conducted *in vitro.* The method of the invention involves lysis of the cells in the biological sample as itaconic acid is an intracellular metabolite.
In another aspect, the invention features the use of the absence, presence and/or level in mammal cells of itaconic acid and/or protein encoded by IRG1, and/or IRG1 expression level as biomarker for inflammation in a method for determining the ability of the cells of a subject to produce itaconic acid under inflammation.
The invention relates to method to determine the ability of a subject to produce itaconic acid under inflammation comprising the steps of:
a. providing a biological sample isolated from said subject;
b. with preference, contacting the biological sample with lipopolysaccharide to induce inflammation;
c. determining, in said biological sample, the absence, the presence and/or the level of at least one molecule selected from the group consisting of : mRNA transcribed from IRG1, cDNA transcribed from IRG1, polypeptide encoded
by IRG1, protein encoded by IRG1, and/or specific fragment thereof; wherein the presence of said at least one molecule is indicative of the ability of said subject to produce itaconic acid under inflammation.

For example, *in vitro* techniques for detection of mRNA include Northern hybridizations and in situ hybridization. *In vitro* techniques for detection of the candidate enzyme include enzyme linked immunosorbent assays (ELISAs), Western blots, immunoprecipitations and immunofluorescence. *In vitro* techniques for detection of candidate cDNA include southern hybridizations. *In vitro* techniques for detection of candidate metabolite (i.e., itaconic acid) include gas chromatography coupled to mass chromatography.

With preference, the molecule selected from the group consisting of polypeptide encoded by IRG1 and/or protein encoded by IRG1 and/or specific fragment thereof, is detected and/or quantified by a method selected from the group consisting of proteonomics, western blot analysis, chromatography, immunoassay, and immunohistochemistry, with preference said immunoassay is selected from the group consisting of ELISA immunoassay and radioimmunoassay.

A preferred agent for detecting and quantifying antibodies for the polypeptide or the protein encoded by IRG1 (i.e. the enzyme catalyzing the production of itaconic acid from cis-aconitate), is an antibody able to bind specifically to this protein or polypeptide, preferably an antibody with a detectable label. Antibodies can be polyclonal or monoclonal antibodies.

Specific monoclonal or polyclonal antibodies for the polypeptide or the protein encoded by IRG1 (i.e. the enzyme catalyzing the production of itaconic acid from cis-aconitate), are available to the skilled man. An isolated enzyme, or a specific fragment thereof can be used as an immunogen to generate antibodies that binds such protein or such polypeptide using standard techniques for polyclonal or monoclonal antibody preparation. It may be also possible to use any fragment of this protein or this polypeptide which contains at least one antigenic determinant to generate these specific antibodies.

The methods described herein may be performed for example by utilizing a kit for determining the ability of cells in a biological sample to produce itaconic acid under inflammation comprising an antibody directed specifically against the peptide encoded by IRG1 or protein encoded by IRG1. With preference said antibody is labeled with a radiolabel, a fluorescent label, a bioluminescent label or a chemiluminescent label.

Preferably, the molecule selected from the group consisting of mRNA or cDNA transcribed from IRG1 and/or specific fragment thereof, is detected using method selected from Northern blot, PCR or RT-PCR.

A preferred agent for detecting and quantifying mRNA or cDNA encoding the protein encoded by IRG1 (i.e. the enzyme catalyzing the production of itaconic acid from cis-aconitate in mammal cells), is a labeled nucleic acid probe or primers able to hybridize this mRNA or cDNA. This nucleic acid probe can be an oligonucleotide of at least 10, 15, 30, 50 or 100 nucleotides length and sufficient to specifically hybridize under stringent conditions to the mRNA or cDNA. The nucleic acid primer can be an oligonucleotide of at least 10, 15 or 20 nucleotides in length and sufficient to specifically hybridize under stringent conditions to the mRNA or cDNA, or complementary sequence thereof.

In certain embodiment of the methods of the present invention, the determination of the absence, presence and/or expression level of IRG1 involves the use of a probe/primer in a polymerase chain reaction (PCR), or alternatively quantitative real time RT-PCR. This method can include the steps of collecting biological sample from a subject, isolating nucleic acid (e.g. mRNA) from the cells of the sample, optionally transforming mRNA into corresponding cDNA, contacting the nucleic acid sample with one or more primers which specifically hybridize to the IRG1 mRNA or the corresponding cDNA under conditions such that hybridization and amplification of IRG1 mRNA or cDNA occurs, and quantifying the presence of the amplification products.

The methods described herein may be performed for example by utilizing a kit for determining the ability of cells in a biological sample to produce itaconic acid under inflammation comprising at least one set of primers capable of amplifying specifically mRNA or cDNA transcribed from IRG1, and/or a set of nucleic probe capable of hybrizing specifically with the mRNA or cDNA transcribed from IRG1. With preference, for IRG1 for which coding sequence is SEQ. ID No. 1, one set of primers used comprises a pair of oligonucleotide primers consisting of the sequences represented by SEQ. ID. Nos. 2 and 3 (wherein A represents adenine, C represents cytosine, G represents guanine and T represents thymine, respectively). The result of PCR is a 96 pb oligonucleotide. With preference, for IRG1 on human chromosome 13, one set of primers used comprises a pair of oligonucleotide primers consisting of the sequences represented by SEQ. ID. Nos. 8 and 9 (wherein A represents adenine, C represents cytosine, G represents guanine and T represents thymine, respectively).

As an embodiment of the methods according to the invention, itaconic acid is detected and/or quantified by metabolomics methods and with preference by gas chromatography coupled mass chromatography.

As further embodiment of the method according all aspects of the invention the biological sample obtained from the subject comprises microglia cells and/or macrophages, with preference the biological sample is selected from the group comprising whole blood, blood serum or plasma, tissue, biopsy, and/or any combination thereof.

The invention also relates to the use of itaconic acid presence and/or level in a biological sample as a biomarker for the diagnosing of inflammation and/or for the determination of the presence in cells of IRG1 and/or protein encoded by IRG1.

In an embodiment of the invention, the above kits also comprise instructions to proceed with the method according to the invention, and/or consumables like LPS to induce inflammation in the cells and/or a lysis buffer to lyse the cells. With preference the lysis buffer has Tris-HCl, EDTA, EGTA, SDS, deoxycholate or any combination thereof.

A pharmaceutical composition for use in preventing or treating inflammation comprising an expression vector containing IRG1 as the active ingredient, with preference IRG1 coding sequence is NCBI Reference Sequence: MM_008392 and correspond to SEQ. ID. No. 1.

In a preferred embodiment of the invention, inflammation is the biological response to pathogens or infectious agent, preferably to bacteria. Therefore, IRG1 can be used in gene therapy in relation with bacterial infection. Therefore the invention relates to a pharmaceutical composition for use in preventing or treating bacterial infection by inducing itaconic acid production comprising an expression vector containing IRG1 as the active ingredient, with preference IRG1 coding sequence is SEQ. ID No. 1.

The invention relates to the use of IRG1 for preparing a pharmaceutical composition to initiate production of itaconic acid in cells of a mammal subject in response to inflammation. Also the invention discloses the use of a IRG1 gene for preparing a pharmaceutical composition to induce production of itaconic acid in the cells of a subject for preventing or treating inflammation or bacterial infection, with preference IRG1 coding sequence is SEQ. ID No. 1.

The above pharmaceutical composition is preferably used in a method of performing gene therapy in a mammal subject, with preference in a human subject, such that gene therapy results in human cells producing itaconic acid under inflammation, with preference the gene therapy results in human macrophage or microglia cells producing itaconic acid under inflammation or in response to bacterial infection.

The presence of IRG1, relevant for production of itaconic acid, can be advantageously used as an identification marker responsive to therapy for inflammation or for bacterial infection.

Therefore the invention relates to the use of a vector transformed with IRG1 for preparing a pharmaceutical composition for use in a method of performing gene therapy in a mammal subject, comprising:
a- transfecting *in vivo* cells selected form the group consisting of macrophage and/or microglia cells, with an effective amount of said vector;
b- allowing said cells to take up the vector, and
c- measuring regression of inflammation.

### Examples

The invention now being generally described, it will be more readily understood by reference to the following examples, which are included merely for the purpose of illustration of certain aspects and embodiments of the present invention, and are not intended to limit the invention.

### Legends of the Figures

- Figure 1a: Heat map showing 43 differential metabolites in 6h LPS (10 ng/ml) treated mouse macrophages (RAW264.7 cell line) relative to untreated macrophages (p<0.05).
- Figure 1b: Heat map showing 91 differential metabolites in RAW264.7 murine macrophages treated for 6 h with LPS (10 ng/ml) relative to untreated macrophages (Welch's t-test, p<0.05).
- Figure 2: Itaconic acid quantification (mM) in mouse microglia cells (BV2 cell line) and mouse macrophages (RAW264.7 cell line) under 6h LPS (10 ng/ml) exposure (in black: untreated cells; in grey: LPS treated cells (6h)).
- Figure 3: Itaconic acid measurement in mouse primary microglia treated during 6h with LPS (1 ng/ml) (in black: untreated cells; in grey: LPS treated cells (6h)).
- Figure 4a: Suggested pathway for production of itaconic acid in A. terreus.
- Figure 4b: TCA cycle scheme with IRG1 enzyme having CAD activity
- Figure 5: Labelling of itaconic acid (grey) and citrate (black) if glucose is used as a tracer in RAW264.7 macrophages.
- Figure 6: IRG1 gene expression and itaconic acid measurement in cells treated with LPS (10 ng/ml) in transfected and untransfected cells.
- Figure 7: Western-blot analysis in the same conditions in RAW264.7 cells (BV2 and THP1 cells are used respectively as positive and negative controls).
- Figure 8a and figure 8b: IRG1 gene expression in mouse primary microglia cells (figure 8a) and in TNF-α primary microglia (figure 8b), treated with LPS (1 ng/ml) for 6h.
- Figure 8c and figure 8d: IRG1 gene expression in BV-2 cells (figure 8c) and in TNF-α primary microglia (figure 8d), treated with LPS (1 ng/ml) for 6h.
- Figure 8e and figure 8f: IRG1 gene expression in RAW264.7 cells (figure 8e) and in TNF-α primary microglia (figure 8f), treated with LPS (1 ng/ml) for 6h.
- Figure 9: pCMV6-Entry Vector
- Figure 10: Gain of function experiment in human A549 lung cancer cells transfected with the overexpressing IRG1 plasmid (pIRG1).
- Figure 11: Itaconic acid and IRG1 signalling pathway as an antimicrobial mechanism.
- Figure 12: Influence on the uptake of S. *pyogenes* by transfected (siRNA IRG1) mouse macrophages (RAW264.7 cell line) after 1h, 2h and 4h of incubation. Results are shown as average bacteria per ml (±SD).
- Figure 13: Purification of cis-aconitate decarboxylase from HEK293T cells transfected with a pCMV6-Entry-IRG1 expression plasmid.
- Figure 14a and 14b: Influence of IRG1 mRNA levels on the antimicrobial activity of macrophages.
- Figure 15: *Salmonella enterica* serovar Typhimurium growth curve.
- Figure 16a and 16b: IRG1 expression and itaconic acid production in human PBMCs-derived macrophages.
- Figure 17: TNF-α expression in LPS-activated human PBMCs-derived macrophages.
- Figure 18: parallel between urea cycle and TCA cycle both producing antimicrobial compounds

### Materials and methods

### Primary mouse microglia

Mixed glial cell cultures were prepared from the brains of new born C57BL/6 mice. After carefully removing meninges and large blood vessels, the brains were pooled and then minced in cold phosphate buffered saline (PBS) solution. The tissue was mechanically dissociated with Pasteur pipettes and the resultant cell suspension was passed through a 21G hypodermic needle. After washes and centrifugations, the mixed glial cells were plated into poly-D-lysine (PDL, Sigma) coated 6-well plates (2 brains per 6-well plate) in Dulbecco's modified Eagle's medium (DMEM) (Invitrogen) supplemented with 100 U/ml penicillin, 100 mg/ml streptomycin (Sigma) and 10% heat-inactivated foetal bovine serum (FBS, Invitrogen) in a water-saturated atmosphere containing 5% CO₂ at 37°C. The medium was replaced every 3-4 days. After 7-10 days, when the cultures reached confluence, microglia were detached by a 30 min shaking on a rotary shaker (180 rpm). Detached cells, mainly microglia (>95%), were then plated in multi-well plates in conditioned medium and further incubated for 3 days.

### Primary human monocytes

Primary human monocytic CD14+ cells were isolated in two steps from blood samples provided by Red Cross Luxembourg. First, peripheral blood mononuclear cells (PBMCs) were separated in 50 ml Leucoseptubes (Greiner) through Ficoll-PaqueTM Premium (GE Healthcare) density-gradient centrifugation at 1000 g for 10 minutes at room temperature with no brake. Second, CD14+ cells were purified with magnetic labeling. Therefore, 2 µl of CD14 Microbeads (Miltenyi Biotech) per 10⁷ PBMCs were incubated for 30 min at 4 °C followed by a positive LS column (Miltenyi Biotech) magnetic selection. The purified CD14+ cells were differentiated in six-well plates for 11 days in RPMI1640 medium without L-glutamine and phenol red (Lonza) supplemented with 10% human serum (A&E Scientific), 1% penicillin/streptomycin (Invitrogen) and L-glutamine (Invitrogen). The medium was changed at day 3 and 7.

### Cell lines

Four cell lines were used for the experiments, specifically :
- murine microglial BV-2 cells (kindly provided by Dr. Djalil Coowar, AxoGlia Therapeutics),
- murine macrophages RAW264.7 (ATCC TIB-71),
- human epithelial A549 lung cancer cells (ATCC CCL-185) and
- human HEK293T cells.

BV-2, HEK293T and RAW264.7 cell lines were maintained in DMEM with or without sodium pyruvate, supplemented with 10% heat-inactivated FBS (South American, Invitrogen). No antibiotics were used for BV-2, 1% penicillin/streptomycin were used for RAW264.7 and HEK293T cells.

A549 cells were cultivated in DMEM without sodium pyruvate, supplemented with 10% heat-inactivated FBS and 1% penicillin/streptomycin. Cells were grown and maintained according to standard cell culture protocols and kept at 37°C with 5% CO₂.

For experiments, BV-2, RAW264.7 and A549 cells were seeded into multi-well plates at a density of 0.5 x 10⁵ (BV-2) and 1.0 x 10⁵ (RAW264.7 and A549) cells/well (six-well plates). After 3 days of culture, the cells were activated adding specific stimuli to the culture medium.

**Lipopolysaccharide** (LPS 055:B5 from *Escherichia coli*, Sigma) was added at different doses for primary microglia (1 ng/ml) and BV2, RAW264.7 or A549 (10 ng/ml) cells to obtain equivalent activation because of the increased sensitivity of the primary cultures compared to the cell lines.

### Gas chromatography/mass spectrometry (GC/MS) sample preparation and procedure

Cells grown on 6-well plates were washed with 1 ml saline solution and quenched with 0.4 ml -20°C methanol. After adding an equal volume of 4°C cold water cells were collected with a cell scraper and transferred in tubes containing 0.4 ml -20°C chloroform. The extracts were vortexed at 1400 rpm for 20 min at 4°C and centrifuged at 16000 g for 5 min at 4°C. 0.3 ml of the upper aqueous phase was collected in specific GC glass vials and evaporated under vacuum at -4°C using a refrigerated CentriVap Concentrator (Labconco). The metabolite extractions of cells grown on 12-well plates were performed using half of the volumes.

The interphase was centrifuged with 1ml -20°C methanol at 16000 g for 5 min at 4°C. The pellet was used for RNA isolation.

Metabolite derivatization was performed using an Agilent Autosampler. Dried polar metabolites were dissolved in 15 µl of 2% methoxyamine hydrochloride in pyridine at 45°C. After 30 minutes an equal volume of MSTFA (2,2,2-trifluoro-N-methyl-N-trimethylsilyl-acetamide) + 1% TMCS (chloro-trimethyl-silane) were added and hold for 30 min at 45°C. Metabolites extracted out of 12-well plates were derivatized using half of the reagent volumes.

GC/MS analysis was performed using an Agilent 6890 GC equipped with a 30 m DB-35MS capillary column. The GC was connected to an Agilent 5975C MS operating under electron impact (EI) ionization at 70 eV. The MS source was held at 230°C and the quadrupole at 150°C. The detector was operated in scan mode and 1 µl of derivatized sample was injected in splitless mode. Helium was used as carrier gas at a flow rate of 1 ml/min. The GC oven temperature was held on 80°C for 6 min and increased to 300°C at 6°C/min. After 10 minutes the temperature was increased to 325°C at 10°C/min for 4 min. The run time of one sample was 59 min.

### Protein purification and CAD activity assay

HEK293T cells were extracted 48 hours after transfection by scraping them into a lysis buffer containing 25 mM Hepes, pH 7.1 and 1x protease inhibitor cocktail (Roche). After two freeze/thaw cycles, cell extracts were incubated for 30 min on ice in the presence of DNAse I (200 U/ml extract; Roche Applied Science) and 10 mM MgSO₄. The crude cell extracts were centrifuged for 5 min at 16000 x *g* (4°C) and pellets were resuspended in lysis buffer for SDS-PAGE analysis. Flag-IRG1 was purified from the supernatant using the Flag®M purification kit, according to the manufacturer's instructions (Sigma Aldrich). About 3 mg protein were loaded onto 250 µl anti-Flag affinity resin and retained proteins were eluted with a solution containing 200 µg/ml Flag peptide (3 x 400 µl fractions). Protein purity was checked by SDS-PAGE analysis. Protein concentration was measured by the Bradford assay using Bradford reagent (Bio-Rad).

Cis-aconitate decarboxylase activity was measured by incubating cell extracts or purified protein fractions (10 µl) at 30°C and for 40 min in a reaction mixture containing 25 mM Hepes, pH 7.1 and 1 mM cis-aconitate in a total volume of 100 µl. Reactions were stopped by addition of 900 µl methanol/water (8:1) mix. After 10 min centrifugation at 13200 rpm and 4°C, 100 µl of the supernatant were collected in specific GC glass vials and evaporated under vacuum at -4°C using a refrigerated CentriVapConcentrator (Labconco). The dry residue was derivatized and itaconic acid was quantified by GC-MS as described below. Itaconic acid concentrations were calculated by comparing the corresponding GC/MS signals obtained in samples with the one obtained after injection of known amounts of itaconic acid standards.

### RNA isolation and reverse-transcription PCR (RT-PCR)

Total RNA was purified from cultured cells using the Qiagen RNeasy Mini Kit (Qiagen) as per manufacturer's instructions. First strand cDNA was synthesized from 0.5-2 µg of total RNA using Superscript III (Invitrogen) with 1 µl (50µM) / reaction oligo(dT)₂₀ as primer. Individual 20 µl SYBR Green real-time PCR reactions consisted of 2 µl of diluted cDNA, 10 µl of 2X iQ™ SYBR Green Supermix (Bio-Rad), and 0.5 µl of each 10 µM optimized forward and reverse primers in 7 µL RNase-free water. Primers sequences have been designed using Beacon Designer software (Bio-Rad), provided by Eurogentec, or directly designed by Thermo Scientific. Sequence forward primer (5'-3') IRG1 is SEQ. ID. No. 2. SEQ. ID. No. 2. binds IRG1 SEQ. No. 1 on nucleotide number 52 to 72. Sequence reverse primer (5'-3') IRG1 is SEQ. ID. No. 3. SEQ. ID. No. 3. binds IRG1 SEQ. No. 1 on nucleotide number 128 to 147. Known primer sequences where used for TNF-α (Mus musculus tumor necrosis factor, referenced NCBI Locus ID NM_0013693). Known primer sequences were used for the housekeeping gene L27 (Mus musculus ribosomal protein L27 referenced NCBI Locus ID NM_0011289). The PCR was carried out on a Light Cycler 480 (Roche Diagnostics), using a 3-stage program provided by the manufacturer: 10 min at 95°C and 40 cycles of 30 sec at 95°C, 30 sec at 60°C, 30 sec at 72°C followed by 10 sec 70-95° melting curves. All experiments included three no-template controls and were performed on three biological replicates with three technical replicates for each sample. For standardization of quantification, L27 was amplified simultaneously.

### SDS-PAGE and Western immunoblotting

Cells were cultivated in Petri's dishes, lysed with 500 µl M-PER® Mammalian Protein Extraction Reagent (Thermo Scientific) supplied with 1x protease inhibitor cocktail (Roche). Protein concentration was measured with a BCA protein assay kit. Heat-denatured samples were separated on 10% SDS-polyacrylamide gels electrophoresis (Bio-Rad) followed by transfer to nitrocellulose membranes 0.2 µm (Sigma). After blocking with 5% (w/v) dry milk in 0.5% Tween 20 in TBS (TBST), the membrane was incubated overnight in primary antibody against IRG1 (Sigma) in 5% BSA/TBST with constant shaking. After three washes with TBST, the membrane was incubated with streptavidin-HRP. The housekeeping control was detected with antibody against β-action (Sigma) and HRP-conjugated donkey anti-rabbit antibody (Westburg). Secondary antibodies were detected with chemiluminescence reagent and band images were captured using the Odyssey 2800 (Licor).

### Statistical analysis

The data shown are the means ± SEM of three experiments. Statistical significance was estimated with Student *t* test for unpaired observations. A *p* value of <0.05 was considered significant

### Ethics Statement

All animal procedures have been performed according to the European Guidelines for the use of animals in research (86/609/CEE). New born mice were decapitated and dissected for the different experiments. All efforts were made to minimize suffering. All animals have been raised and crossed in an indoor animal house in a 12 h light/dark cycle and have been provided with water and food *ad libitum.*

### EXAMPLE I

### Identification of itaconic acid in mammalian cells

This example describes the presence of itaconic acid in mammalian cells under LPS activation using metabolomics profile of resting and activated macrophage and microglia cells.

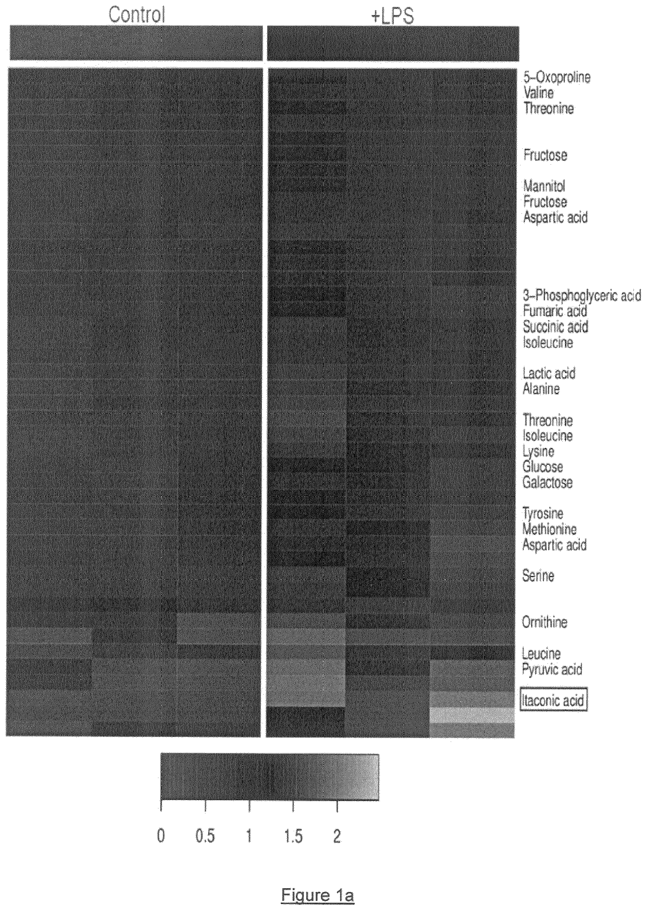

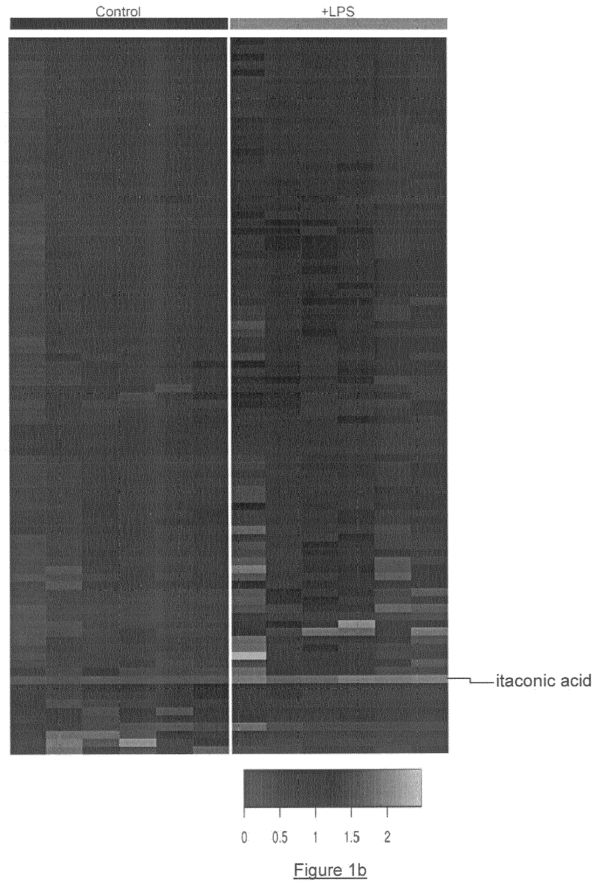

The cells have been lysed with addition of a lysis buffer and submitted to metabolite extraction. Metabolite extraction was performed by addition of a mixture of methanol, water and chloroform. Metabolites were detected by gas chromatography coupled to mass chromatography. Similar extraction and metabolite detection was performed on untreated macrophages. A comparative heat map with a subset of 43 metabolites detected with respect to RAW264.7 cell lines, illustrated in figure 1a, demonstrates the presence of itaconic acid in mouse macrophage, and in particular in LPS treated macrophages. A comparative heat map showing 91 differential metabolites in RAW264.7 murine macrophages treated for 6 h with LPS (10 ng/ml) relative to untreated macrophages (Welch's t-test, p<0.05) is illustrated in figure 1 b. A total of 293 intracellular metabolites were detected across all samples and 91 showed significantly different levels. The metabolite most significantly affected by LPS stimulation was identified as itaconic acid (*p*=2.5x10⁻⁸).

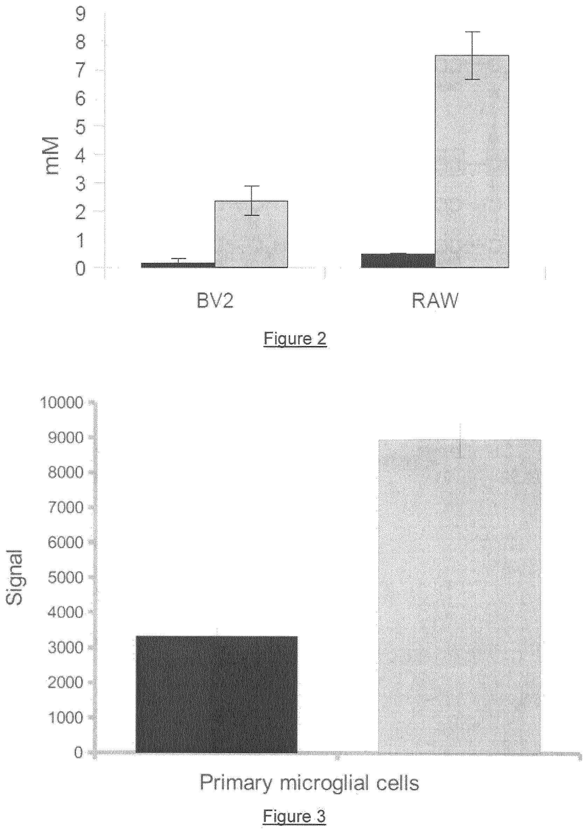

Experiments were conducted in order to quantify the production of itaconic acid in mammal cells. Metabolite extraction and quantification was performed on mouse microglia cells (BV2 cell line) and mouse macrophages (RAW264.7 cell line) under 6h LPS (10 ng/ml) exposure and in parallel on untreated mouse microglia cells (BV2 cell line) and mouse macrophages (RAW264.7 cell line). Results are shown on figure 2a were untreated cells are illustrated in black and LPS treated cells (6h) in grey. Untreated cells show an itaconic acid concentration of less than 1 mM, whereas treated cells show higher concentrations. LPS treated BV2 cells show an itaconic acid concentration of more than 2 mM, and LPS treated RAW264.7 cells show a concentration of more than 7 mM. The results demonstrate the production of itaconic acid upon LPS exposure by mouse microglia cells and mouse macrophages.

Similar results were found in murine primary microglial cells under the same pro-inflammatory conditions. Figure 3 shows GCMS results identifying the presence of itaconic acid in LPS treated cells (in grey) which show a higher level than untreated cells (in black). These findings clearly point towards a potential immunological function of this metabolite.

Itaconic acid production by mouse macrophage and microglia cells is induced upon in times of LPS exposure, and at high levels. According to these results itaconic acid can be a biomarker of LPS induced inflammation or more generally of inflammation in cells. It has been established that concentration of itaconic acid in mouse macrophage or microglia cells of more than 0,6 mM, with preference of more than 1 mM, with preference of more than 2 mM is indicative of cells being inflamed. With preference, itaconic acid concentration of more than 1 mM in microglia cells is indicative of the presence of an inflammation. With preference, itaconic acid concentration of more than 2 mM and preferably of more than 5 mM in macrophages is indicative of the presence of an inflammation.

From the experimental data, it has been established inflammation can be detected in a subject by comparison of itaconic acid concentration measured in microglia cells and/or macrophage of the subject with reference itaconic acid concentration. With the reference itaconic acid concentration is measured in control microglia cells and/or macrophage from a subject presenting no inflammation. According to the invention, inflammation is detected when the ratio between measured itaconic acid concentration and itaconic acid reference concentration is more than 2, preferably more than 5, and preferably more than 8.

### EXAMPLE II

### Itaconic acid production in the tricarboxylic acid (TCA) cycle

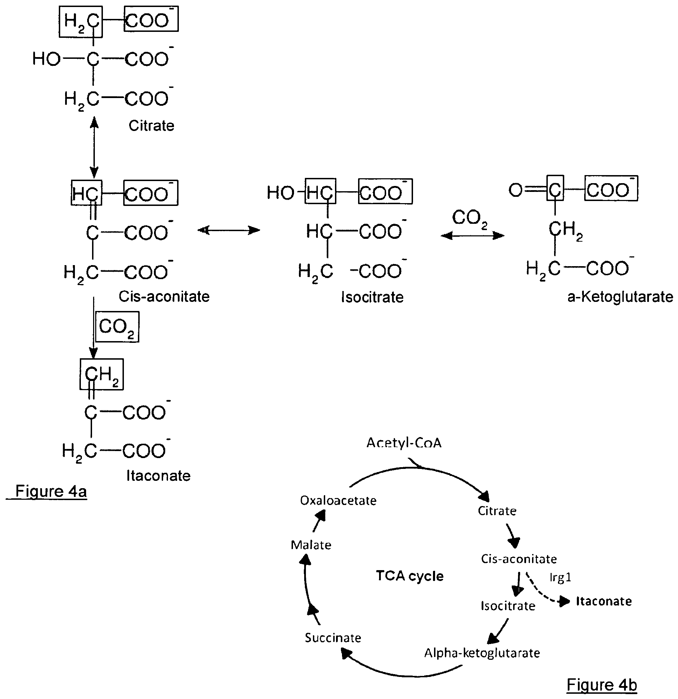

Figure 4a describes the suggested pathway of production of itaconic acid in *Aspergillus terreus.* Atoms coming from gycolysis are marked. The decarboxylation of cis-aconitate to itaconate is done by the cis-aconitate decarboxylase. From this suggested pathway, itaconate can only contain one labeled carbon if produced in the first round of the TCA cycle (figure 4b).

To test if itaconic acid production follows a similar pathway in mammal cells, labeled glucose (with labeled carbon) was used as tracer in RAW264.7 macrophages. Therefore, LPS-activated RAW264.7 macrophages were incubated with uniformly ¹³C-labeled glucose (U-¹³C₆). Citrate synthase catalyzes the transfer of two labeled carbon atoms from acetyl-CoA to oxaloacetate resulting in M2 cis-aconitate isotopologues. If the decarboxylation is performed by a CAD homologue, the first carbon atom of the molecule will be decarboxylated resulting in M1 isotopologues of itaconic acid.

To perform glucose labeling assay, RAW264.7 macrophages were seeded at a density of 1 x 10⁶ per well in 12-well plates in DMEM medium supplemented with 10% FBS and 1% penicillin/streptomycin at 37°C with 5% CO₂. After 24 hours, the medium was changed to DMEM containing uniformly labeled 25 mM [U-¹³C] glucose (Cambridge Isotope). Simultaneously, the cells were activated with 10 ng/ml LPS. After 6 h of incubation, the metabolites were extracted.

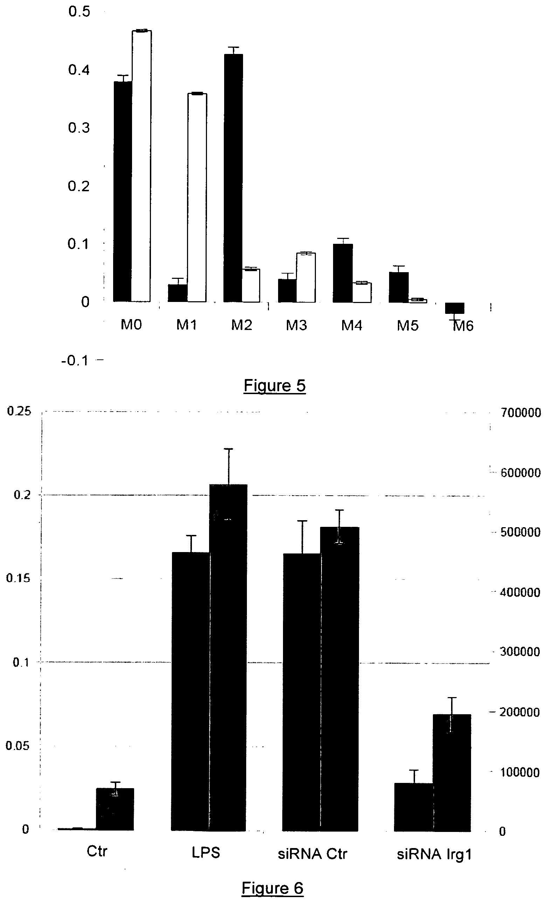

The results shown in figure 5 indicate the production of labeled itaconate (in grey) as well as labeled citrate (in black). It can be seen that 45% of the citrate molecules as M2 isotopologues whereas 38% of the itaconic acid molecules were M1 isotopologues. A significant fraction of M2, M3 and M4 itaconic acid isotopologues was found. The M4 fraction of itaconic acid reflects pyruvate carboxylase or reverse malic enzyme activity. Due to the symmetry of succinate, subsequent turns of the TCA cycle can result in M2 or M3 isotopologues of itaconic acid. Similar results demonstrating itaconic acid production in mammalian cells have been recently reported in C.L. Strelko et al.," Itaconic acid is a mammalian metabolite induced during macrophage activation". J. Am. Chem. Soc. 133, 16386 (2011).

The major fraction of labeled itaconate contains one istope whereas citrate contains mainly two labeled atoms. This demonstrates that the suggested pathway of production in Aspergillus terreus may be similar to the pathway in macrophages.

### EXAMPLE III

### Association of IRG1 with itaconic acid production

This example describes the IRG1 gain and loss of function experiments that associate IRG1 expression with itaconic acid production.

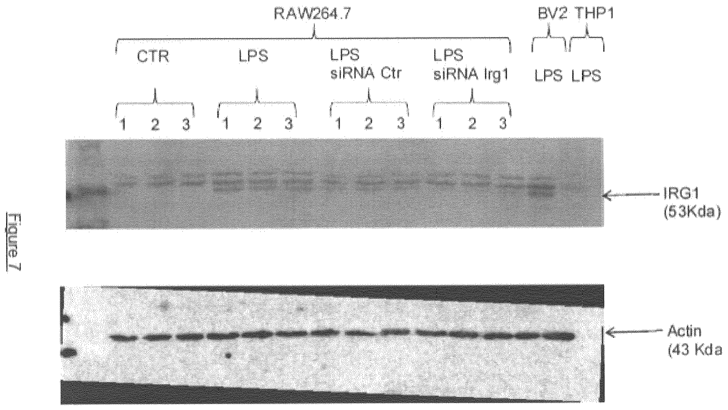

Figure 7 show Western-blot analysis in the same conditions in RAW264.7 cells (BV2 and THP1 cells are used respectively as positive and negative controls). This demonstrates that the protein is encoded by IRG1 as it is found when the gene is activated and not found when the gene is silenced. The absence of protein in THP1 confirms that itaconic acid is not produced in human cells.

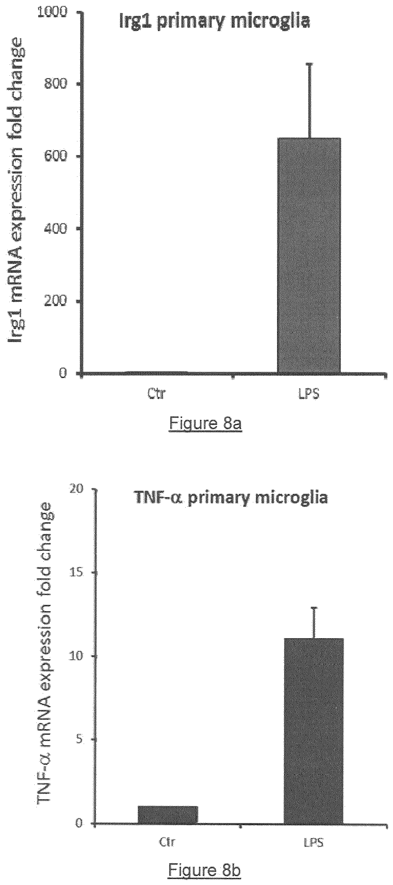

Figures 8a and 8b show, respectively, IRG1 and TNF-α gene expression in mouse primary microglia cells, treated with LPS (1 ng/ml) for 6h.

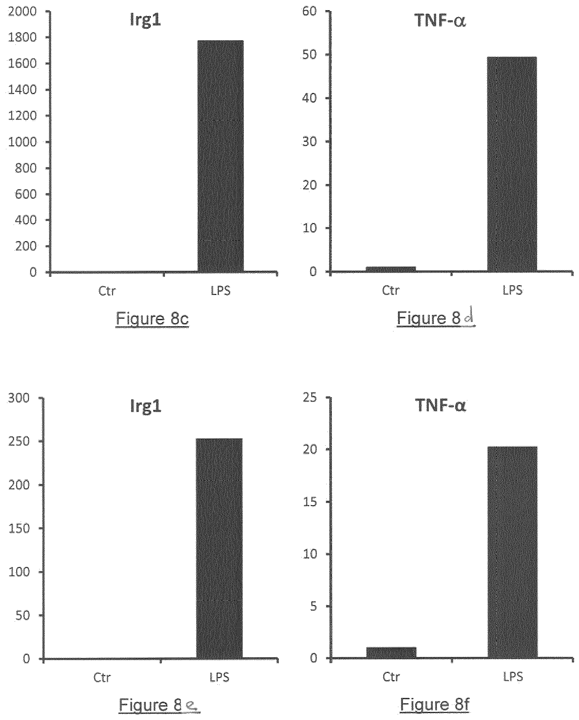

Figures 8c and 8d show, respectively, IRG1 and TNF-α gene expression in BV-2 cells, treated with LPS (1 ng/ml) for 6h.
Figures 8e and 8f show, respectively, IRG1 and TNF-α gene expression in RAW264.7 cells, treated with LPS (1 ng/ml) for 6h.

### Cells transfections

Human A549 lung cancer cells were tranfected with the overexpressing IRG1 plasmid (plIRG1). In parallel GFP plasmid was transfected into A549 lung cancer cells as control. Itaconic acid signal was measured.

The ON-TARGETplus SMARTpool containing four different siRNA sequences, all specific to murine IRG1 (siRNA IRG1), and the corresponding non-targeting control (siRNA Ctr) were designed and synthesized by Thermo Scientific Dharmacon. The sequences of specific siRNAs are given SEQ. ID. No. 4 to. SEQ. ID. No. 7, and SEQ. ID. No. 10 (wherein A represents adenine, C represents cytosine, G represents guanine and U represents uracil, respectively).

RAW264.7 macrophages were transfected with Amaxa Nucleofection Device (Amaxa), using the Amaxa SG cell line 4D Nucleofector Kit according to the manufacturer's instructions.

Briefly, transfection with siRNA complexes was carried out from pelleted and resuspended 1 x 10⁶ cells per condition. Transfection reagent and siRNA were prepared according to manufacturer's instructions (Amaxa). Final dosing concentrations of siRNAs provided per each condition were 100 nM. After the nucleofection processing using "RAW264.7 program" on the Nucleofection Device, the cells were seeded at a density of 1 x 10⁶ cells per well in 12-well plates in DMEM supplemented with 10% FBS at 37°C with 5% CO2 during 24h.

pCMV6-IRG1 overexpressing plasmid (4 µg, *Mus musculus* immune responsive gene 1 transfection-ready DNA, OriGene), in parallel with the GFP plasmid (4 µg), was transfected into A549 cells using Lipofectamine 2000 (Invitrogen) and further incubated for 24h. pCMV6-Entry-IRG1 plasmid was transfected into HEK293T cells by the jetPEI procedure as described previously (*22*) and further incubated for 48 h before extraction.

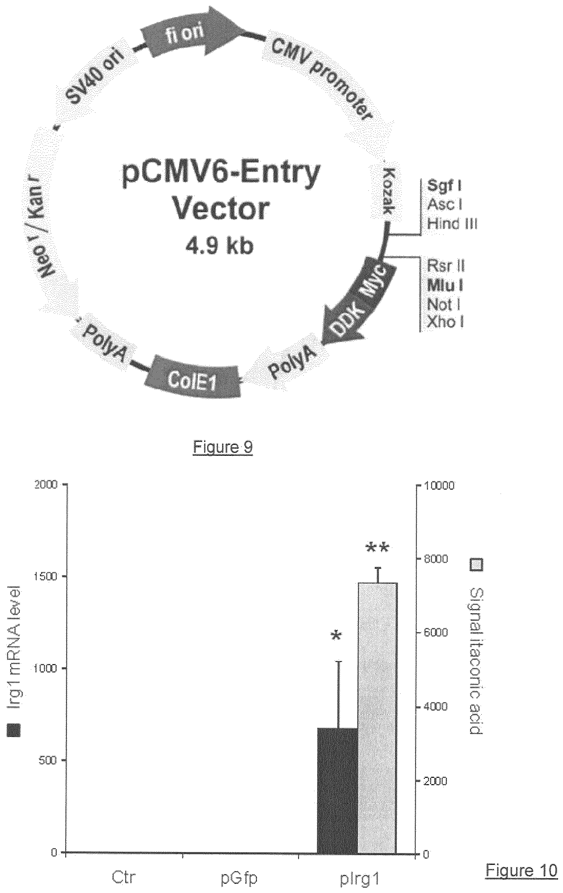

The vector used is shown in figure 9. The results shown in figure 10 demonstrate a gain of function for cells transfected with IRG1 plasmid.

### RNA interference

Further evidence for the physiological role of IRG1 in itaconic acid production, RNA interference was employed to selectively silence the expression of this gene in RAW264.7 cells.

Figure 6 shows the results of IRG1 gene expression and itaconic acid measurement in cells treated with LPS (10 ng/ml) in transfected and untransfected cells. Metabolites and RNA extractions were realized after 6h of incubation. Real-time RT-PCR results are normalized using L27 as housekeeping gene and are shown as average expression fold change (±SEM) relative to IRG1 mRNA in control. The silencing of IRG1 resulted in an 80% decrease of IRG1 mRNA and a 60% decrease of itaconic acid concentration in LPS-activated macrophages. Very low levels of both IRG1 mRNA (17-fold less when compared to LPS activated cells) and itaconic acid (11.5-fold less) were detected in non-activated macrophages. The correlation between the metabolite level and the RNA level demonstrates the association of IRG1 with itaconic acid production.

In line with the stable-isotope labeling results, these experiments indicate a link between this enzyme and the TCA cycle.

### Purification of cis-aconitate decarboxylase

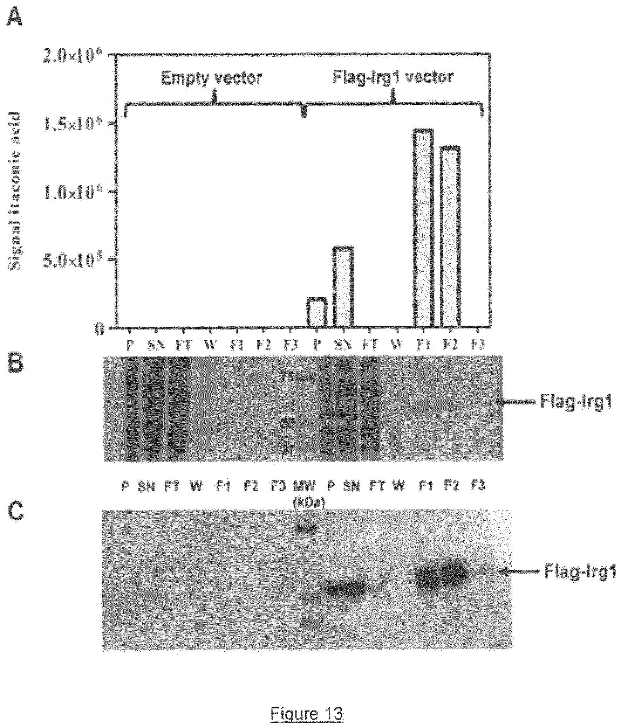

To directly demonstrate that the IRG1 protein catalyzes cis-aconitate decarboxylation, FLAG-tagged protein was purified from HEK293T cells transfected with a pCMV6-Entry-IRG1 expression plasmid. Figure 13a shows extracts from cells transfected with empty plasmid or Flag-IRG1 plasmid were loaded onto an affinity resin (Cell MM2, FlagM purification kit, Sigma Aldrich) and proteins were eluted with Flag peptide. Cis-aconitate decarboxylase activity was measured in cell extracts and purification fractions. As depicted in Figure 13A, extracts prepared from those cells catalyzed the conversion of cis-aconitate to itaconic acid. No itaconic acid formation was detected when extracts prepared from cells transfected with empty vector were incubated with cis-aconitate. Furthermore, affinity purification of the extract prepared from Flag-IRG1 overexpressing cells clearly showed coelution of the cis-aconitate decarboxylase activity with a protein band identified as IRG1 by SDS-PAGE (expected MW ∼ 55 kDa for Flag-IRG1; Fig. 13B) and Western blot analysis using anti-IRG1 antibody (Fig. 13C). SDS-PAGE analysis showed that this purification procedure yielded a virtually homogenous preparation of the IRG1 protein (Fig. 13B) thus demonstrating that the cis-aconitate decarboxylase activity measured in the purified fractions was not due to contaminating protein. A CAD specific activity of about 40 nmol min⁻¹ mg protein⁻¹ was measured in the peak activity fraction.

In figure 13B: 12 µl of each protein fraction was loaded onto an SDS-PAGE gel that was stained with Coomassie Blue. Precision plus protein kaleidoscope standards from Bio-Rad were used for molecular weight estimation.

In figure 13C: Western Blot analysis of the same protein fractions was performed using an IRG1 specific antibody.

In figure 13 the following abbreviations have been used: P= pellet; SN= supernatant; FT= flow through; W= wash; F1-F3= elution fractions.

### EXAMPLE IV

### Itaconic acid production in the cell associated with antimicrobial activity

This example describes the influence of intracellular itaconic acid concentration on the growing of bacteria, assessing the suggested pathway as an antimicrobial mechanism.

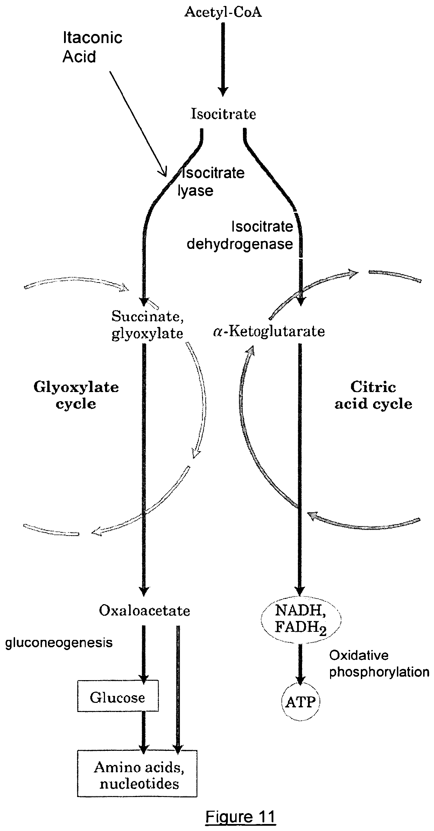

The glyoxylate cycle (Figure 11) is mobilized when bacteria are grown on fatty acids as the limiting carbon source. The glyoxylate shunt is the first step leading to the flux of carbon into gluconeogenesis, which is the only mechanism by which the organism can acquire and retain carbon from fatty acids.

It has been previously demonstrated that itaconic acid inhibits the bacterial isocytrate lyase (ICL) (T.R. Patel, B.A. McFadden, Exp. Parasitol. 44, 262 (1978*) ;* B.A. McFadden, S. Purohit, J. Bacteriol. 131, 136 (1977*)),* an enzyme of the glyoxylate shunt essential for bacterial survival during growth on fatty acids or acetate as the limiting carbon source (S. Hillier, W.T. Charnetzky, J. Bacteriol. 145, 452 (1981); S.L. Hillier, W.T. Charnetzky, J. Clin. Microbiol. 13, 661 (1981*)).* Furthermore, it has been shown that *Mycobacterium tuberculosis* cannot persist in macrophages when both isoforms of ICL are genetically knocked out (E.J. Muñoz-Elias, J.D. McKinney, Nat. Med. 11, 638 (2005*)*)*.* As the glyoxylate shunt is exclusively found in prokaryotes, lower eukaryotes and plants, it affords a unique target for drug development.

Bacterial phagocytosis and killing assay were conducted to test the influence of the uptake of *S*. *pyogenes* by transfected (siRNA IRG1) mouse macrophages (RAW264.7 cell line) after 1 h, 2h and 4h of incubation.

Transfected RAW264.7 macrophages (with unspecific siRNA or IRG1 specific siRNA) were infected with *Streptococcus pyogenes* strain A20 at a multiplicity of infection (MOI) of 10:1 (10 bacteria per macrophage) and incubated for 1h at 37°C, 5% CO2. Macrophages were then washed with sterile PBS, resuspended in complete medium containing 100 mg/ml gentamicin to kill non ingested bacteria and further incubated for 2h at 37°C, 5% CO2. Macrophages were then disrupted with dH2O to release intracellular bacteria (this was considered time 0h relative to gentamicin treatment) or 1, 2, 4h later (this was considered time 1, 2, 4h relative to gentamicin treatment) and the amount of viable intracellular bacteria was determined by plating on blood agar.

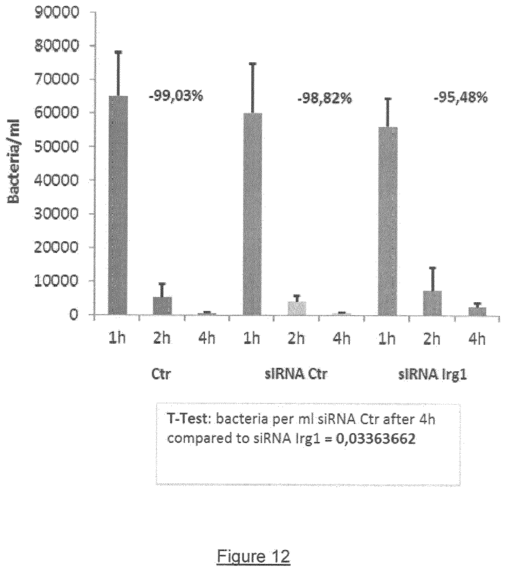

Results are shown as average bacteria per ml (±SD). The results reported on figure 12 demonstrate that when IRG1 is silenced the macrophages antimicrobial activity after 4 hours is reduced. This demonstrates that the reduction of their antimicrobial activity is related to the inhibition of IRG1 expression, thus associated to the decrease of itaconic acid concentration in macrophages.

### EXAMPLE V

### Involvement of IRG1-mediated synthesis of itaconic acid in the antimicrobial activity of the macrophages

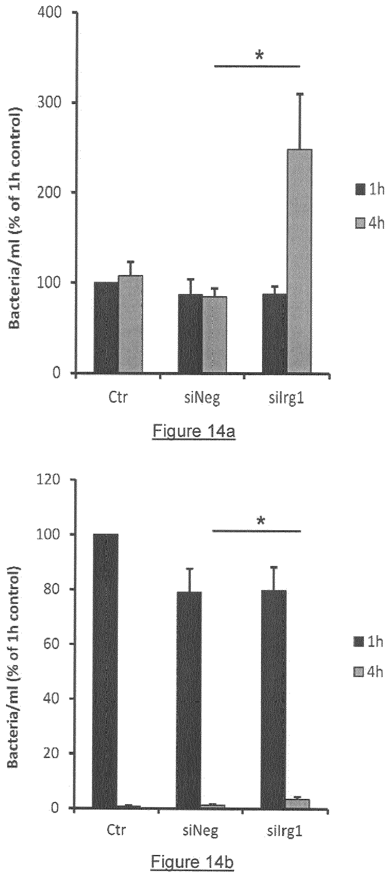

Macrophages were infected with two different pathogens. Figures 14a and b relates to the influence of IRG1 mRNA levels on the antimicrobial activity of macrophages.

RAW264.7 cells were transfected with either siRNA specific for IRG1 or with siRNA Ctr. Macrophages were infected with *Salmonella enterica* serovar Typhimurium at a multiplicity of infection of 0.1 bacteria per macrophages (in figure 14a) or *Streptococcus pyogenes* at a multiplicity of infection of 10 bacteria per macrophages (in figure 14b). In both cases, infections were performed after 24 h of transfection and incubated for 1 h or 4 h at 37°C. Macrophages were then resuspended in medium containing 100 mg/ml gentamicin to kill non-ingested bacteria and further incubated for 2 h. Macrophages were then disrupted with H₂O to release intracellular bacteria and the amount of viable intracellular bacteria was determined by plating on blood agar. Bars represent numbers of bacteria per ml (±SEM). *p-value<0.05.

### Salmonella enterica serovar Typhimurium

RAW264.7 macrophages were infected with *Salmonella enterica* serovar Typhimurium, a facultative intracellular pathogen previously shown to require ICL for persistent infection in mice. Silencing IRG1 gene significantly impaired the ability of macrophages to inhibit the growth of these bacteria. Indeed, a significantly larger number of intracellularly viable bacteria were detected in macrophages treated with siRNA targeting IRG1 compared to those treated with an unspecific control siRNA 4 h after infection (Fig. 14a).

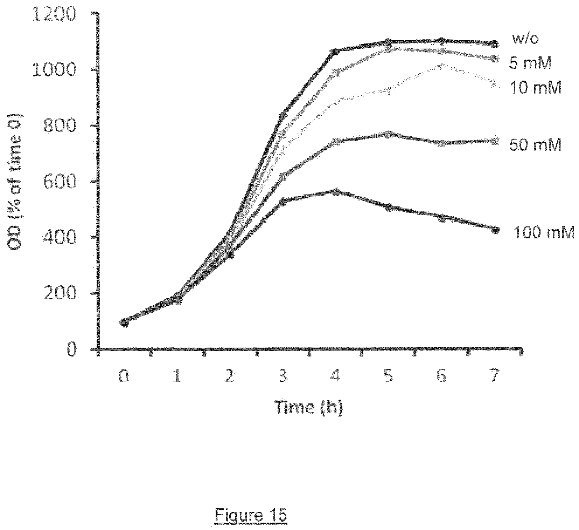

To directly investigate the effect of itaconic acid on bacterial replication, *S*. *enterica* was grown in liquid minimal medium supplemented with acetate as the unique carbon source and various concentrations of itaconic acid. The latter exerted a dose-dependent growth inhibition with a significant effect observed for concentrations higher than10 mM. Figure 15 shows *Salmonella enterica* serovar Typhimurium growth curve. S. *enterica* was grown in liquid medium with acetate as unique carbon source in the presence of increasing concentrations of itaconic acid (5, 10, 50, 100 mM). The optical density (OD) was measured every hour to record the bacterial growth. Curves are calculated in % relative to time 0 and represent the mean of three independent experiments. These observations demonstrate an antimicrobial effect of itaconic acid against *Salmonella* possibly by inhibiting ICL function.

### Streptococcus pyoge

Macrophages were also infected with *Streptococcus pyogenes,* an extracellular pathogen that does not possess an ICL enzyme and has previously been shown to be efficiently killed by macrophages (O. Goldmann, M. Rohde, G.S. Chhatwal, E. Medina, Role of macrophages in host resistance to group A streptococci. Infect. Immun. 72, 2956 (2004*)).* Although almost all bacteria were killed by macrophages 4 h after infection, a significantly larger number of intracellular viable bacteria was detected in macrophages treated with siRNA targeting IRG1 when compared to those treated with unspecific siRNA (Fig. 14b). However, the antimicrobial effect of itaconic acid was less pronounced in streptococci than in Salmonella. Taken together, these results clearly demonstrate the importance of the IRG1 enzyme in macrophages during infection.

### EXAMPLE VI

### IRG1 expression and acid itaconic levels in human immune cells

A gene sequence homology based investigations revealed an IRG1 homologue in the fungus *Aspergillus terreus.* The mouse IRG1 and the *A. terreus* cis-aconitate decarboxylase (CAD), an enzyme converting cis-aconitate into itaconic acid, share a 23% amino acid sequence identity.

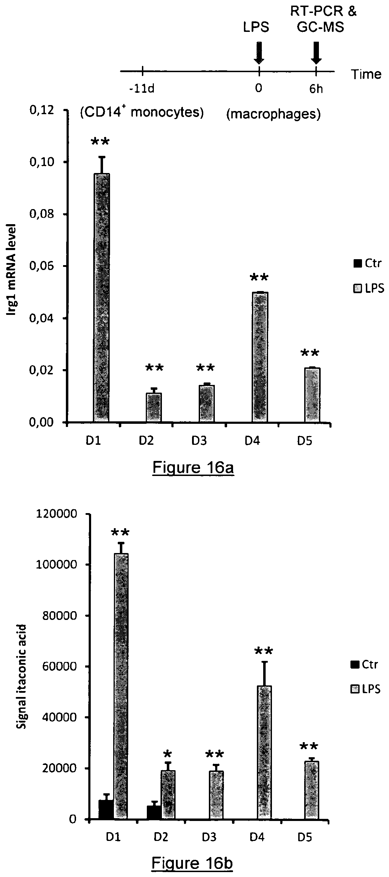

Since an IRG1 homologous gene is annotated in the human genome on chromosome 13 (fig. 16), IRG1 expression and itaconic acid levels were analyzed in human immune cells.

Figure 16a and b illustrate IRG1 expression and itaconic acid production in human PBMCs-derived macrophages. Figure 16a relates to levels of mRNA and figure 16b relates to itaconic acid in resting (Ctr) or LPS-activated (10µg/ml) PBMCs-derived macrophages from five different donors (D). RNA and metabolites extractions were performed after 6 h of stimulation.

In figure 16a, the levels of IRG1 mRNA were determined by real-time RT-PCR and normalized using L27 as housekeeping gene. Each bar represents the average expression fold change of three replicates (±SEM).

In figure 16b, the levels of itaconic acid were determined by GC/MS measurements. Each bar represents itaconic acid levels (±SEM). *p-value<0.05, **p-value<0.01.

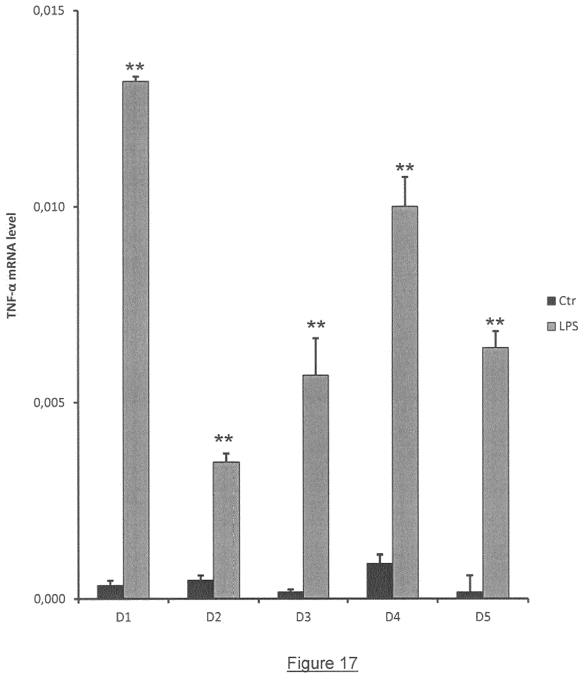

In line with the observations in mouse macrophages, IRG1 expression in human peripheral blood mononuclear cells (PBMCs)-derived macrophages was highly up-regulated under LPS activation compared to resting conditions where IRG1 mRNA levels were almost undetectable (Fig. 16a). These results are in accordance with those of Roach and colleagues (J.C. Roach et al., Transcription factor expression in lipopolysaccharide-activated peripheral blood-derived mononuclear cells. Proc. Natl. Acad. Sci. USA 104, 16245 (2007)), who analyzed LPS-activated PBMCs transcriptional profile observing IRG1 up-regulation compared to control conditions. At the metabolite level, itaconic acid amounts were highly increased under LPS-induced inflammatory conditions compared to resting cells where the metabolite was measured in low amounts or below detection limits (Fig. 16b). The differences at the transcriptional level between the analyzed donors correlated with the amounts of intracellular itaconic acid found in activated macrophages of each specific donor. These differences, in relation with results of figure 17, reflect variability in the ability of each individual to react to an inflammation.

Figure 17 shows TNF-α expression in LPS-activated human PBMCs-derived macrophages. RNA extractions were performed after 6 h of LPS (10 µg/ml) stimulation of PBMCs-derived macrophages from five different donors (D). The levels of TNF-α mRNA were determined by real-time RT-PCR and normalized using L27 as housekeeping gene. Each bar represents the average expression fold change of three replicates (±SEM). **p-value<0.01.

### Conclusion

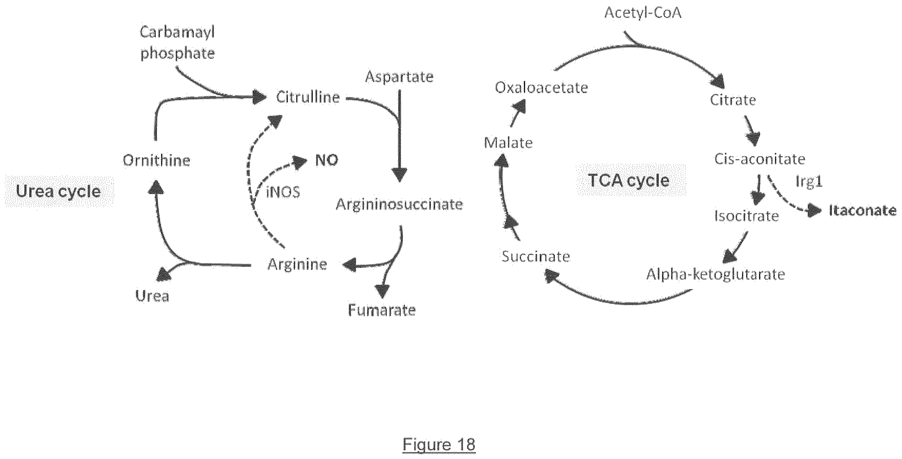

Figure 18 is a parallel between urea cycle and TCA cycle both producing antimicrobial compounds. Under inflammatory conditions, inducible nitric oxide synthase (iNOS) and IRG1 expression are both up-regulated, thus catalyzing the production of nitric oxide (NO) and itaconic acid, respectively.

The discovery of an inducible enzyme linked to the TCA cycle and catalyzing the production of an antimicrobial metabolite is reminiscent of the mechanism found in the urea cycle responsible for the production of nitric oxide (NO). In this case, the inducible nitric oxide synthase (iNOS) is the enzyme that catalyzes the production of NO from L-arginine (fig. 18). iNOS, like IRG1, is induced in mouse microglial cells under pro-inflammatory conditions and catalyzes the synthesis of the antimicrobial compound NO in these cells. NO and itaconic acid production by immune cells thus seem to represent an intrinsic property of these cells to self-react towards an inflammatory insult. The antimicrobial agents NO and itaconic acid seem to confer a form of metabolic immunity to macrophages or microglial cells.

In summary, the inventors demonstrated that the IRG1 gene codes for an enzyme synthesizing itaconic acid from the TCA cycle intermediate cis-aconitate. Furthermore, they showed a strong upregulation of both IRG1 transcript and itaconic acid synthesis in macrophages in response to an inflammatory insult. The data also provide evidence that itaconic acid contributes to the antimicrobial activity of macrophages. The results of this study reveal a previously unknown role of the TCA cycle to mediate metabolic immunity in mammalian immune cells.

## Claims

1. A method for predicting the presence of IRG1 and/or protein encoded by IRG1 in a subject, comprising the steps of:
a- providing a biological sample isolated from said subject
b- determining the presence and/or the level of itaconic acid in a biological sample,
wherein the presence and/or the level of itaconic acid is indicative of the presence of IRG1 and/or of the protein encoded by IRG1, in at least a part of the cells of said biological sample.

2. The method according to claim 1 **characterized in that** the biological sample of step (a) is contacted with lipopolysaccharide in order to initiate production of itaconic acid before the step (b) of determining the presence and/or the level of itaconic acid in the biological sample.

3. A method to determine the ability of a human subject, to react to inflammation, comprising the step of:
a. providing a biological sample isolated from said subject
b. contacting said biological sample with lipopolysaccharide
c. determining the presence and/or the level of itaconic acid in a biological sample,
wherein the presence and/or the level of itaconic acid is indicative of the ability of the subject to react to inflammation.

4. A method to determine the ability of a subject to produce itaconic acid under inflammation comprising the steps of:
a. providing a biological sample isolated from said subject;
b. with preference, contacting the biological sample with lipopolysaccharide to induce inflammation;
c. determining, in said biological sample, the absence, the presence and/or the level of at least one molecule selected from the group consisting of: mRNA transcribed from IRG1, cDNA transcribed from IRG1, polypeptide encoded by IRG1, protein encoded by IRG1, and/or specific fragment thereof, wherein said fragment has at least one of the functional characteristics or property of IRG1;
wherein the presence of said at least one molecule is indicative of the ability of said subject to produce itaconic acid under inflammation.

5. The method according to claim 4 **characterized in that** the molecule selected from the group consisting of: polypeptide encoded by IRG1, protein encoded by IRG1, and/or specific fragment thereof, wherein said fragment has at least one of the functional characteristics or property of IRG1 is detected and/or quantified by a method selected from the group consisting of proteonomics, western blot analysis, chromatography, immunoassay, and immunohistochemistry, with preference said immunoassay is selected from the group consisting of ELISA immunoassay and radioimmunoassay.

6. The method according to claim 4 **characterized in that** the molecule selected from the group consisting of mRNA transcribed from IRG1, cDNA transcribed from IRG1, and/or specific fragment thereof, wherein said fragment has at least one of the functional characteristics or property of IRG1, is detected and/or quantified by a method selected from the group consisting of Northern blot, PCR and RT- PCR.

7. A method for detecting inflammation in a human subject, comprising the step of determining the presence and/or the level of itaconic acid in a biological sample isolated from said subject wherein the presence and/or level of itaconic acid is indicative of the presence of inflammation.

8. A method for detecting pro-inflammatory condition in macrophages of a human subject comprising the step of determining the presence and/or the level of itaconic acid in a biological sample isolated from said subject wherein the presence and/or the level of itaconic acid is indicative of a proinflammatory condition.

9. The method according to claims 1 to 8 **characterized in that** the biological sample obtained from the subject comprises microglia cells and/or macrophages, with preference the biological sample is selected from the group comprising whole blood, blood serum or plasma, tissue, biopsy, and/or any combination thereof.

10. Use of itaconic acid presence and/or level in a biological sample as a biomarker for the determination of the presence in cells of IRG1 and/or protein encoded by IRG1.

11. Use of itaconic acid presence and/or level in a biological sample comprising human cells, such as macrophage and/or microglia cells, as a biomarker for inflammation, with preference in a method for identifying a compound candidate for pharmacological agent in the treatment of inflammation.

12. A kit for determining the ability of cells in a biological sample from a human subject to produce itaconic acid under inflammation **characterized in that** it comprises at least one selected from: a set of primers capable of amplifying specifically mRNA or cDNA transcribed from IRG1, wherein the set of primers comprise a pair of oligonucleotide primers consisting of the sequences represented by SEQ. ID. Nos. 2 and 3 or by SEQ. ID Nos. 8 and 9.

13. The use of claim 11 to be performed with a kit for determining the ability of cells in a biological sample from a human subject to produce itaconic acid under inflammation, wherein the kit is **characterized in that** it comprises at least one antibody directed specifically against the peptide encoded by IRG1 or protein encoded by IRG1, wherein the antibody is labeled with a radiolabel, a florescent label a bioluminescent label or a chemiluminescent label.

14. A pharmaceutical composition for use in preventing or treating inflammation or bacterial infection by inducing itaconic acid production comprising an expression vector containing IRG1 as the active ingredient, with preference IRG1 coding sequence is SEQ. ID No. 1.

## Patentansprüche

1. Verfahren zum Vorhersagen des Vorhandenseins von IRG1 und/oder von Protein, das durch IRG1 kodiert wird, in einem Subjekt, umfassend die Schritte:
a. Bereitstellen einer aus dem Subjekt isolierten biologischen Probe,
b. Feststellen des Vorhandenseins von und/oder des Levels an Itaconsäure in einer biologischen Probe,
wobei das Vorhandensein von und/oder das Level an Itaconsäure indikativ für das Vorhandensein von IRG1 und/oder von Protein, das durch IRG1 kodiert wird, in zumindest einem Teil der Zellen der biologischen Probe ist.

2. Verfahren nach Anspruch 1, **dadurch gekennzeichnet, dass** die biologische Probe von Schritt (a) mit Lipopolysaccharid in Kontakt gebracht wird, um die Produktion von Itaconsäure vor dem Schritt (b) des Feststellens des Vorhandenseins von und/oder des Levels an Itaconsäure in der biologischen Probe auszulösen.

3. Verfahren zum Feststellen des Vermögens eines menschlichen Subjekts auf Entzündung zu reagieren, umfassend die Schritte:
a. Bereitstellen einer aus dem Subjekt isolierten biologischen Probe,
b. Inkontaktbringen der biologischen Probe mit Lipopolysaccharid,
c. Feststellen des Vorhandenseins von und/oder des Levels an Itaconsäure in einer biologischen Probe,
wobei das Vorhandensein von und/oder das Level an Itaconsäure indikativ für das Vermögen des Subjekts ist auf Entzündung zu reagieren.

4. Verfahren zum Feststellen des Vermögens eines Subjekts bei Entzündung Itaconsäure zu produzieren, umfassend die Schritte:
a. Bereitstellen einer aus dem Subjekt isolierten biologischen Probe;
b. mit Präferenz, Inkontaktbringen der biologischen Probe mit Lipopolysaccharid, um eine Entzündung auszulösen;
c. Feststellen, in der biologischen Probe, das Fehlen, das Vorhandensein und/oder das Level mindestens eines Moleküls, das aus der Gruppe ausgewählt wird, die aus mRNA, die von IRG1 transkribiert wurde, cDNA, die von IRG1 transkribiert wurde, Polypeptid, das durch IRG1 kodiert wird, Protein, das durch IRG1 kodiert wird und/oder einem spezifischen Fragment davon, wobei das Fragment mindestens eines der funktionellen Merkmale oder Eigenschaften von IRG1 aufweist, besteht;
wobei das Vorhandensein des mindestens einen Moleküls indikativ für das Vermögen des Subjekts ist, bei Entzündung Itaconsäure zu produzieren.

5. Verfahren nach Anspruch 4, **dadurch gekennzeichnet, dass** das Molekül, das aus der Gruppe ausgewählt wird, die aus Polypeptid, das durch IRG1 kodiert wird, Protein, das durch IRG1 kodiert wird und/oder einem spezifischem Fragment davon, wobei das Fragment mindestens eines der funktionellen Merkmale oder Eigenschaften von IRG1 aufweist, besteht, nachgewiesen wird und/oder quantifiziert wird mit einem Verfahren, das aus der Gruppe ausgewählt wird, die aus Proteonomik, Western-Blot-Analyse, Chromatographie, Immunoassay und Immunhistochemie besteht, mit Präferenz ist der Immunoassay ausgewählt aus der Gruppe, die aus ELISA-Immunoassay und Radioimmunoassay besteht.

6. Verfahren nach Anspruch 4, **dadurch gekennzeichnet, dass** das Molekül aus der Gruppe ausgewählt wird, die aus mRNA, die von IRG1 transkribiert wird, cDNA, die von IRG1 transkribiert wird und/oder einem spezifischen Fragment davon, wobei das Fragment mindestens eines der funktionellen Merkmale oder Eigenschaften von IRG1 aufweist, besteht, nachgewiesen und/oder quantifiziert wird mit einem Verfahren, das aus der Gruppe ausgewählt wird, die aus Northern Blot, PCR und RT-PCR besteht.

7. Verfahren zum Feststellen einer Entzündung bei einem menschlichen Subjekt, umfassend den Schritt des Feststellens des Vorhandenseins von und/oder des Levels an Itaconsäure in einer aus dem Subjekt isolierten biologischen Probe, wobei das Vorhandensein von und/oder das Level an Itaconsäure indikativ für das Vorliegen einer Entzündung ist.

8. Verfahren zum Feststellen eines präinflammatorischen Zustandes bei Makrophagen eines menschlichen Subjekts, umfassend den Schritt des Feststellens des Vorhandenseins von und/oder des Levels an Itaconsäure in einer aus dem Subjekt isolierten biologischen Probe, wobei das Vorhandensein von und/oder das Level an Itaconsäure indikativ für einen präinflammatorischen Zustand ist.

9. Verfahren nach einem der Ansprüche 1 bis 8, **dadurch gekennzeichnet, dass** die von dem Subjekt erhaltene biologische Probe Mikrogliazellen und/oder Makrophagen umfasst, mit Präferenz ist die biologische Probe aus der Gruppe ausgewählt, die aus Vollblut, Blutserum oder-plasma, Gewebe, Biopsie und/oder einer Kombination davon besteht.

10. Verwendung des Vorhandenseins von und/oder des Levels an Itaconsäure in einer biologischen Probe als Biomarker zur Feststellung des Vorhandenseins von IGR1 und/oder von Protein, das durch IRG1 kodiert wird, in Zellen.

11. Verwendung des Vorhandenseins von und/oder des Levels an Itaconsäure in einer biologischen Probe, umfassend menschliche Zellen, wie Makrophagen- und/oder Mikrogliazellen, als Biomarker für Entzündung, mit Präferenz in einem Verfahren zur Ermittlung eines Verbindungskandidaten für einen pharmakologischen Wirkstoff zur Behandlung einer Entzündung.

12. Kit zum Feststellen des Vermögens von Zellen in einer biologischen Probe von einem menschlichen Subjekt bei Entzündung Itaconsäure zu produzieren, **dadurch gekennzeichnet, dass** es umfasst: mindestens eines ausgewählt aus: einem Set von Primern, die imstande sind, von IRG1 transkribierte mRNA oder cDNA spezifisch zu amplifizieren, wobei das Set von Primern ein Paar von Oligonucleotid-Primern, bestehend aus den durch SEQ ID Nr. 2 und SEQ ID Nr. 3 oder durch SEQ ID Nr. 8 und SEQ ID Nr. 9 dargestellten Sequenzen, umfasst.

13. Verwendung nach Anspruch 11, durchzuführen mit einem Kit zum Feststellen des Vermögens von Zellen in einer biologischen Probe von einem menschlichen Subjekt bei Entzündung Itaconsäure zu produzieren, wobei das Kit **dadurch gekennzeichnet ist, dass** es mindestens einen Antikörper, spezifisch gerichtet gegen das Peptid, das durch IRG1 kodiert wird oder das Protein, das durch IRG1 kodiert wird, umfasst, wobei der Antikörper mit einem radioaktiven Marker, einem Fluoreszenzmarker, einem Biolumineszenzmarker oder einem Chemilumineszenzmarker markiert ist.

14. Pharmazeutische Zusammensetzung zur Verwendung bei der Vorbeugung oder Behandlung von Entzündung oder bakterieller Infektion durch Auslösen der Itaconsäureproduktion, umfassend einen Expressionsvektor, der IRG1 als Wirkstoff enthält, mit Präferenz ist die IRG1 kodierende Sequenz SEQ ID Nr. 1.

## Revendications

1. Un procédé pour prédire la présence de IRG1 et/ou de protéine codée par IRG1 chez un sujet, comprenant les étapes consistant à :
a. fournir un échantillon biologique isolé à partir dudit sujet
b. déterminer la présence et/ou le niveau d'acide itaconique dans un échantillon biologique,
dans lequel la présence et/ou le niveau d'acide itaconique indique la présence de IRG1 et/ou de la protéine codée par IRG1, dans au moins une partie des cellules dudit échantillon biologique.

2. Le procédé selon la revendication 1, **caractérisé en ce que** l'échantillon biologique de l'étape (a) est mis en contact avec du lipopolysaccharide afin d'initier la production d'acide itaconique avant l'étape (b) déterminant la présence et/ou le niveau d'acide itaconique dans l'échantillon biologique.

3. Un procédé pour déterminer la capacité d'un sujet humain, à réagir à une inflammation, comprenant l'étape consistant à :
a. fournir un échantillon biologique isolé à partir dudit sujet
b. mettre en contact ledit échantillon biologique avec du lipopolysaccharide
c. déterminer la présence et/ou le niveau d'acide itaconique dans un échantillon biologique,
dans lequel la présence et/ou le niveau d'acide itaconique indique la capacité du sujet à réagir à une inflammation.

4. Un procédé pour déterminer la capacité d'un sujet à produire de l'acide itaconique sous inflammation comprenant les étapes consistant à :
a. fournir un échantillon biologique isolé à partir dudit sujet ;
b. avec de préférence, mise en contact de l'échantillon biologique avec du lipopolysaccharide pour induire une inflammation ;
c. déterminer, dans ledit échantillon biologique, l'absence, la présence et/ou le niveau d'au moins une molécule choisie dans le groupe constitué par : de l'ARNm transcrit à partir de IRG1, de l'ADNc transcrit à partir de IRG1, un polypeptide codé par IRG1, une protéine codée par IRG1 et/ou un fragment spécifique de ceux-ci, ledit fragment ayant au moins l'une des caractéristiques fonctionnelles ou une propriété de IRG1 ;
dans lequel la présence de ladite au moins une molécule indique l'aptitude dudit sujet à produire de l'acide itaconique sous inflammation.

5. Le procédé selon la revendication 4, **caractérisé en ce que** la molécule choisie dans le groupe constitué de : un polypeptide codé par IRG1, une protéine codée par IRG1, et/ou un fragment spécifique de ceux-ci, ledit fragment ayant au moins l'une des caractéristiques fonctionnelles ou une propriété de IRG1, est détectée et/ou quantifiée par un procédé choisi dans le groupe constitué de protéomique, analyse de type western blot, chromatographie, dosage immunologique et immunohistochimie, ledit dosage immunologique étant de préférence choisi dans le groupe constitué par un dosage immunologique et un dosage radio-immunologique de type ELISA.

6. Le procédé selon la revendication 4, **caractérisé en ce que** la molécule, choisie dans le groupe constitué par de l'ARNm transcrit à partir de IRG1, de l'ADNc transcrit à partir de IRG1 et/ou un fragment spécifique de ceux-ci, dans lequel ledit fragment a au moins l'une des caractéristiques fonctionnelles ou une propriété de IRG1, est détectée et/ou quantifiée par un procédé choisi dans le groupe constitué de Northern blot, PCR et RT-PCR.

7. Un procédé pour détecter une inflammation chez un sujet humain, comprenant l'étape consistant à déterminer la présence et/ou le niveau d'acide itaconique dans un échantillon biologique isolé à partir dudit sujet, dans lequel la présence et/ou le niveau d'acide itaconique indique la présence d'une inflammation.

8. Un procédé pour détecter un état pro-inflammatoire dans les macrophages d'un sujet humain, comprenant l'étape consistant à déterminer la présence et/ou le niveau d'acide itaconique dans un échantillon biologique isolé à partir dudit sujet, dans lequel la présence et/ou le niveau d'acide itaconique indique un état pro-inflammatoire.

9. Le procédé selon les revendications 1 à 8, **caractérisé en ce que** l'échantillon biologique obtenu à partir du sujet comprend des cellules microgliales et/ou des macrophages, l'échantillon biologique étant de préférence choisi dans le groupe comprenant le sang total, du sérum ou du plasma sanguin, un tissu, une biopsie, et/ou toute combinaison de ceux-ci.

10. Utilisation de la présence et/ou du niveau d'acide itaconique dans un échantillon biologique en tant que biomarqueur pour la détermination de la présence dans les cellules de IRG1 et/ou de protéine codée par IRG1.

11. Utilisation de la présence et/ou du niveau d'acide itaconique dans un échantillon biologique comprenant des cellules humaines, telles que des macrophages et/ou des cellules microgliales, en tant que biomarqueur pour l'inflammation, de préférence dans un procédé pour identifier un composé candidat pour un agent pharmacologique dans le traitement de l'inflammation.

12. Une trousse permettant de déterminer la capacité de cellules dans un échantillon biologique provenant d'un sujet humain à produire de l'acide itaconique sous inflammation, **caractérisé en ce qu'**elle comprend au moins un élément choisi parmi : un ensemble d'amorces capables d'amplifier spécifiquement l'ARNm ou l'ADNc transcrit à partir de IRG1, dans laquelle l'ensemble d'amorces comprennent une paire d'amorces d'oligonucléotides constitués des séquences représentées par les SEQ. ID. No 2 et 3 ou par les SEQ. ID No 8 et 9.

13. L'utilisation selon la revendication 11 à réaliser avec une trousse permettant de déterminer la capacité de cellules dans un échantillon biologique provenant d'un sujet humain à produire de l'acide itaconique sous inflammation, dans laquelle la trousse est **caractérisée en ce qu'**elle comprend au moins un anticorps dirigé spécifiquement contre le peptide codé par IRG1 ou la protéine codée par IRG1, dans laquelle l'anticorps est marqué par un radiomarqueur, un marqueur fluorescent, un marqueur bioluminescent ou un marqueur chimiluminescent.

14. Une composition pharmaceutique utilisée pour prévenir ou traiter une inflammation ou une infection bactérienne par induction de la production d'acide itaconique, comprenant un vecteur d'expression contenant IRG1 comme principe actif, la séquence codant IRG1 étant de préférence la SEQ. ID No 1.


### SEQUENCE LISTING

<110> Université du Luxembourg
<120> Method to predict the presence of inflammation or itaconic Acid, IRG1 and/or protein IRG1 in a subject and pharmaceutical composition for treating or preventing inflammation
<130> PT02086WO
<160> 7
<210> 1
   <211> 2588
   <212> DNA
   <213> Mus musculus
<400> 1
   ccagccagca actactcctg ccatccactc ctgagccagt taccctccag agcaacatga 60
   tgctcaagtc tgtcacagag agctttgctg gtatgattca cggcttgaaa gtgaaccacc 120
   tgacagatgg tatcattcgg aggagcaaga ggatgatcct ggattctctg ggcgttggct 180
   tcctggggac aggcacagaa gtgttccata aagtcaccca atatagtaaa atctacagtt 240
   ccaacacctc cagcactgtt tggggtcgac cagacttcag gctcccaccg acatatgctg 300
   cttttgttaa tggtgttgct gttcactcca tggattttga tgacacatgg caccctgcca 360
   cccacccttc tggggctgtc ctacctgtcc tcacagctct atcggaagcc ctgcctcaga 420
   ctcccaagtt ttctggcctc gacctgctgc tggcgttcaa cgttggtatt gaagtacagg 480
   gacgattaat gcacttctcc aaggaagcca aagacatacc aaagagattc caccctccct 540
   ctgtggtggg gactctggga agtgctgctg ctgcgtccaa gtttctgggg ctcagcttga 600
   caaagtgccg cgaggcattg gctattgctg tttcccacgc aggggcaccc atagcgaacg 660
   ctgccactca gactaagccc cttcatattg gcaatgcagc caagcatggg atggaagcca 720
   cgtttctggc aatgctgggc ctccaaggaa acaaacagat cttggacctg gggtcagggt 780
   tcggtgcctt ctatgccaac tactcccccg aagaccttcc aagcctggat tctcacatct 840
   ggctgttgga ccagcaggat gtggccttta agagcttccc ggcacatctg gctacccact 900
   gggtggcaga tgcagctgca gccgtgagaa agcaccttgt gacaccagaa agagccctgt 960
   tccctgctga ccacatcgag agaatcgtgc tcaggatccc tgacgtccag tacgtaaaca 1020
   ggcccttccc ggactcagag catgaagccc gtcattcttt ccagtatgtg gcctgtgcct 1080
   cgctgctcga cggtagcatc actgtcccat ccttccacag ccagcaggtc aataggcctc 1140
   aggtgagaga gttgctcaag aaggtgaagc tggagcatcc tcctgacaac ccgccaagct 1200
   tcgacacgct atactgtgaa ataagcatca ctctaaagga cgggaccact ttcaccgagc 1260
   gctctgacac cttctatggt cactggagga aaccactgag ccaggaagat ctgcgcaaca 1320
   agttccgagc caatgcctca aagatgctat gcagggacac ggtggaaagc cttataacgg 1380
   tagtagaaaa gctagaagac ctagaagact gctctgtgct aaccagactt ctgaaaggac 1440
   cctctgtcca agatgaagct tcaaaactat ccagcatgtc ctcattcgat cacacaacgt 1500
   tgcccaggtt taccaatatc taaacgacgt tgcactggaa gaaatacatt gttttggttt 1560
   cctgctccac cttcccagca atgagctcag caaagtagag agtccagaaa cagagacaag 1620
   cgtatatgga aagaggcttg tctataattt gataaaaacc agtcctacta ccttcaagat 1680
   atcaaagaaa caaacaacac aggatctgat gtgtatttca taggggtaca gtctaagtca 1740
   cctgttgtct gcttgtaatg ttttctgagg aattatttat taactctggg aacttcacag 1800
   atgtagagga agtggaacgt gctggctggc tgcagatgga gcaagcttgg tctgcccatg 1860
   acttatccag acagggtgcc caaccaaccc ctcttttttt tttctttcca cacagagcct 1920
   taagtgtgta gccaagaatc cttctgctca gcttctctac tgcagatcag acagtttgac 1980
   ttggggtggt gaagcagtgt ggcataagac ccaaggtttc attctctata ttagatagcc 2040
   tgttagctca aaaaaaaaaa gcattttgag aatatttgac aagttgaaga cacaataaac 2100
   atgaaaaggg gaaagttgta aacgtgtcac attagcaacc acgctgaatg tcttctgaat 2160
   ccctagtgtt ctcaagaaaa gaacgggttc taagtcccag atgttgcctg gcgagcatcc 2220
   tcagggaaat agtgtgggta gccatttgac aactccagca agaatatatc agttctcagt 2280
   tcataggagt agaaaatata gttagaaaaa tgataatcca atggaagaaa ggaaaaaaag 2340
   agggagtggt aaacaaagaa aatatgatgt ttggaaatca ccatataaac agaaagaatc 2400
   ttgagtatat ccacagtgcc aacaaacaaa aacaaaagcc aaacaagaaa tgctcctgct 2460
   agctagaatc aagattgtta gaacatgctt cgtttttaaa atgcttagca gttttattga 2520
   tgttttatta atcacccacc tgaagtatac catccaacga catatagtaa atttgtgctc 2580
   acattgcc                                                          2588
<210> 2
   <211> 21
   <212> DNA
   <213> Artificial sequence
<220>
   <223> oligonucleotide primer
<400> 2
   gcaacatgat gctcaagtct g 21
<210> 3
   <211> 20
   <212> DNA
   <213> Artificial sequence
<220>
   <223> oligonucleotide primer
<400> 3
   tgctcctccg aatgatacca 20
<210> 4
   <211> 19
   <212> RNA
   <213> Artificial sequence
<400> 4
   GAAAGUGAAC CACCUGACA 19
<210> 5
   <211> 19
   <212> RNA
   <213> Artificial sequence
<400> 5
   GAGCUUUGCU GGUAUGAUU 19
<210> 6
   <211> 19
   <212> RNA
   <213> Artificial sequence
<400> 6
   GAAAUAAGCA UCACUCUAA 19
<210> 7
   <211> 19
   <212> RNA
   <213> Artificial sequence
<400> 7
   GCACAGAAGU GUUCCAUAA 19
<210> 8
   <211> 24
   <212> DNA
   <213> Artificial sequence
<220>
   <223> oligonucleotide primer
<400> 8
   AGAAGCCCTG CCAAGGAGTC CAAA 24
<210> 9
   <211> 24
   <212> DNA
   <213> Artificial sequence
<220>
   <223> oligonucleotide primer
<400> 9
   CCAGAGCTTC TCGGCACTTT GTCG 24
<210> 10
   <211> 19
   <212> RNA
   <213> Artificial sequence
<400> 10
   GCACAGAAGU GUUCCAUAA 19